# EUROPEAN PATENT SPECIFICATION

(11) **EP 1 827 485 B1**
(45) Date of publication and mention of the grant of the patent: **18.12.2013**
(21) Application number: 05824336.1
(22) Date of filing: 15.12.2005
(51) Int. Cl.: A61K 38/19, A61K 38/20, A61P 17/02, A61P 17/04, A61K 38/21, A61P 17/06, A61P 17/08, A61P 17/10, A61P 17/12

(54) **COMPOSITIONS FOR PREVENTING, REDUCING OR TREATING KERATINOCYTE-MEDIATED INFLAMMATION**
ZUSAMMENSETZUNGEN ZUR PRÄVENTION, VERRINGERUNG ODER BEHANDLUNG VON KERATINOZYTEN-VERMITTELTER ENTZÜNDUNG
COMPOSITIONS DE PREVENTION, DE REDUCTION OU DE TRAITEMENT D'UNE INFLAMMATION INDUITE PAR KERATINOCYTES

(30) Priority: 15.12.2004 EP 04293004
(43) Date of publication of application: 05.09.2007
(73) Proprietor: Université De Poitiers, 86034 Poitiers Cedex (FR); Bioalternatives SAS, 86160 Gencay (FR)
(72) Inventor: LECRON, Jean-Claude, 86300 Bonnes (FR); GASCAN, Hugues, F-49100 Angers (FR); MOREL, Franck, F-86800 Savigny l'Evescault (FR); CHEVALIER, Sylvie, F-49100 Angers (FR); BERNARD, François-Xavier, F-86160 Saint Maurice la Clouere (FR); BONIFACE, Katia, F-86280 Saint Benoit (FR); DIVEU, Caroline, Palo Alto, CA 94306 (US)
(74) Representative: Marcadé, Véronique
(86) International application number: PCT/EP2005/014198
(87) International publication number: WO 2006/063864

(56) References cited:
- WO-A-01/37874
- US-A- 5 744 442
- US-A- 5 958 442
- US-A1- 2002 025 316

### SEQUENCE LISTING

<110> UNIVERSITE D'ANGERS
   UNIVERSITE DE POITIERS
   BIOALTERNATIVES SAS

   LECRON, Jean-Claude
   GASCAN, Hughes
   MOREL, Franck
   CHEVALIER, Sylvie
   BERNARD, François-Xavier
   BONIFACE, Katia
   DIVEU, Caroline
<120> COMPOSITIONS FOR PREVENTING, REDUCING OR TREATING KERATINOCYTE-MEDIATED INFLAMMATION.
<130> VMAahF1872/2WO
<160> 32
<170> PatentIn version 3.3
<210> 1
   <211> 20
   <212> DNA
   <213> Artificial
<220>
   <223> forward primer for reverse transcription of OSMR
<400> 1
   cctgcctacc tgaaaaccag 20
<210> 2
   <211> 20
   <212> DNA
   <213> Artificial
<220>
   <223> reverse primer for reverse transcription of OSMR
<400> 2
   acattggtgc cttcttccac 20
<210> 3
   <211> 22
   <212> DNA
   <213> Artificial
<220>
   <223> forward primer for reverse transcription of gp130
<400> 3
   gggcaatatg actctttgaa gg 22
<210> 4
   <211> 22
   <212> DNA
   <213> Artificial
<220>
   <223> reverse primer for reverse transcription of gp130
<400> 4
   ttcctgttga tgttcagaat gg 22
<210> 5
   <211> 20
   <212> DNA
   <213> Artificial
<220>
   <223> forward primer for reverse transcription of LIFR
<400> 5
   cagtacaaga gcagcggaat 20
<210> 6
   <211> 20
   <212> DNA
   <213> Artificial
<220>
   <223> reverse primer for reverse transcription of LIFR
<400> 6
   ccagtccata aggcatggtt 20
<210> 7
   <211> 20
   <212> DNA
   <213> Artificial
<220>
   <223> forward primer for reverse transcription of GAPDH
<400> 7
   accacagtcc atgccatcac 20
<210> 8
   <211> 20
   <212> DNA
   <213> Artificial
<220>
   <223> reverse primer for reverse transcription of GAPDH
<400> 8
   tccaccaccc tgttgctgta 20
<210> 9
   <211> 28
   <212> DNA
   <213> Artificial
<220>
   <223> forward primer for Q-RT-PCR of S100A7
<400> 9
   gcatgatcga catgtttcac aaatacac 28
<210> 10
   <211> 23
   <212> DNA
   <213> Artificial
<220>
   <223> reverse primer for Q-RT-PCR of S100A7
<400> 10
   tggtagtctg tggctatgtc tcc 23
<210> 11
   <211> 26
   <212> DNA
   <213> Artificial
<220>
   <223> forward primer for Q-RT-PCR of S100A9
<400> 11
   gctcctcggc tttgacagag tgcaag 26
<210> 12
   <211> 30
   <212> DNA
   <213> Artificial
<220>
   <223> reverse primer for Q-RT-PCR of S100A9
<400> 12
   gcatttgtgt ccaggtcctc catgatgtgt 30
<210> 13
   <211> 22
   <212> DNA
   <213> Artificial
<220>
   <223> forward primer for Q-RT-PCR of hBD2/4
<400> 13
   gccatcagcc atgagggtct tg 22
<210> 14
   <211> 21
   <212> DNA
   <213> Artificial
<220>
   <223> reverse primer for Q-RT-PCR of hBD2/4
<400> 14
   aatccgcatc agccacagca g 21
<210> 15
   <211> 20
   <212> DNA
   <213> Artificial
<220>
   <223> forward primer for Q-RT-PCR of KRT10
<400> 15
   gcccgacggtagagttcttt 20
<210> 16
   <211> 21
   <212> DNA
   <213> Artificial
<220>
   <223> reverse primer for Q-RT-PCR of KRT10
<400> 16
   cagaaaccac aaaacacctt g 21
<210> 17
   <211> 20
   <212> DNA
   <213> Artificial
<220>
   <223> forward primer for Q-RT-PCR of OSM
<400> 17
   tcagtctggt ccttgcactc 20
<210> 18
   <211> 20
   <212> DNA
   <213> Artificial
<220>
   <223> reverse primer for Q-RT-PCR of OSM
<400> 18
   ctgcagtgct ctctcagttt 20
<210> 19
   <211> 19
   <212> DNA
   <213> Artificial
<220>
   <223> forward primer for Q-RT-PCR of GAPDH
<400> 19
   gaaggtgaag gtcggagtc 19
<210> 20
   <211> 20
   <212> DNA
   <213> Artificial
<220>
   <223> reverse primer for Q-RT-PCR of GAPDH
<400> 20
   gaagatggtg atgggatttc 20
<210> 21
   <211> 22
   <212> DNA
   <213> Artificial
<220>
   <223> forward primer for Q-RT-PCR of murine S100A8
<400> 21
   tccaatatac aaggaaatca cc 22
<210> 22
   <211> 18
   <212> DNA
   <213> Artificial
<220>
   <223> reverse primer for Q-RT-PCR of murine S100A8
<400> 22
   tttatcacca tcgcaagg 18
<210> 23
   <211> 20
   <212> DNA
   <213> Artificial
<220>
   <223> forward primer for Q-RT-PCR of murine S100A9
<400> 23
   gaaggaattc agacaaatgg 20
<210> 24
   <211> 18
   <212> DNA
   <213> Artificial
<220>
   <223> reverse primer for Q-RT-PCR of murine S100A9
<400> 24
   atcaactttg ccatcagc 18
<210> 25
   <211> 19
   <212> DNA
   <213> Artificial
<220>
   <223> forward primer for Q-RT-PCR of murine MIP-1ß
<400> 25
   cctctctctc ctcttgctc 19
<210> 26
   <211> 20
   <212> DNA
   <213> Artificial
<220>
   <223> reverse primer for Q-RT-PCR of murine MIP-1ß
<400> 26
   agatctgtct gcctcttttg 20
<210> 27
   <211> 18
   <212> DNA
   <213> Artificial
<220>
   <223> forward primer for Q-RT-PCR of murine MDC
<400> 27
   tgctgccagg actacatc 18
<210> 28
   <211> 19
   <212> DNA
   <213> Artificial
<220>
   <223> reverse primer for Q-RT-PCR of murine MDC
<400> 28
   tagcttcttc acccagacc 19
<210> 29
   <211> 20
   <212> DNA
   <213> Artificial
<220>
   <223> forward primer for Q-RT-PCR of murine TARC
<400> 29
   cattcctatc aggaagttgg 20
<210> 30
   <211> 19
   <212> DNA
   <213> Artificial
<220>
   <223> reverse primer for Q-RT-PCR of murine TARC
<400> 30
   cttgggtttt tcaccaatc 19
<210> 31
   <211> 19
   <212> DNA
   <213> Artificial
<220>
   <223> forward primer for Q-RT-PCR of murine GAPDH
<400> 31
   atcaagaagg tggtgaagc 19
<210> 32
   <211> 20
   <212> DNA
   <213> Artificial
<220>
   <223> reverse primer for Q-RT-PCR of murine GAPDH
<400> 32
   gccgtattca ttgtcatacc 20

## Description

The present invention relates to the field of epidermal repair. More particularly, the invention concerns the use of a molecule able to inhibit a heteromeric receptor comprising OSMRβ as a subunit, for the preparation of a composition for inhibiting keratinocyte migration and/or the expression of inflammatory factors by the keratinocytes. The invention also pertains to the use of a molecule which activates heteromeric receptor comprising OSMRβ as a subunit, for obtaining *in vitro* and/or *in vivo* models of inflammatory skin diseases.

The skin is a large and complex tissue providing a protective interface between an organism and its environment. Epidermis forms its external surface, and is mainly constituted of multiple layers of specialized epithelial cells named keratinocytes. Skin can be injured by many different causes, including micro-organisms, chemicals, behaviours, physical injury, ageing, U.V. irradiation, cancer, autoimmune or inflammatory diseases.

Epidermis homeostasis is regulated by a balance between differentiation and proliferation of keratinocytes, differentiating from the basal to the cornified layers of the skin. In response to epidermal stress or in some skin diseases, this equilibrium is broken. Keratinocytes become able to differentially respond to soluble mediators such as Epidermal Growth Factor (EGF) family members, and to additional growth factors and cytokines (FGFs, IGF-1, PDGF, HGF, TGFβ family members, GM-CSF, TSLP, IL-1, TNF-α). These modulators are produced by the keratinocytes themselves, the skin fibroblasts, the Langerhans cells or by immune infiltrating cells such as T lymphocytes. In response, the keratinocytes release additional signaling molecules, modulate the expression level of cell surface receptors, modify their cytoskeleton morphology, and modulate their migration, differentiation and proliferation capacities. These changes are associated with an inflammatory response, leading to either wound healing or to a chronic disease.

Psoriasis is the most common auto-immune disease of the skin, affecting 2 % of the population. The pathology results from a hyperactivation of T lymphocytes that remain resident in the epidermis of psoriatic lesions. Through the secretion of T helper type 1-like cytokines, these T cells contribute to the epidermal proliferation and thickness in psoriatic patients (Lew, Bowcock et al. 2004).

Recently, it has been reported that epidermal keratinocytes from transgenic mice that express constitutively active STAT3 showed up-regulation of several gene products that are linked to the pathogenesis of psoriasis (Sano, Chan et al. 2005). However, although required, STAT3 activation alone is not sufficient to induce psoriatic lesions. The exact nature of the cytokines secreted by activated intralesional T cells involved in keratinocyte alterations, including cellular hyperproliferation, abnormal differentiation and a specific gene expression profile, characteristic of the psoriatic lesion, has not been established.

The cytokines of the IL-6 family are multifunctional proteins regulating cell growth and differentiation in a large number of biological systems, such as immunity, hematopoiesis, neural development, reproduction, bone modeling and inflammatory processes. This cytokine family encompasses nine different members: IL-6, IL-11, IL-27, leukemia inhibitory factor (LIF), cardiotrophin-1, cardiotrophin-like factor, ciliary neurotrophic factor, neuropoietin, and oncostatin M (OSM). The activities of theses cytokines are mediated through ligand-induced oligomerization of a dimeric or trimeric receptor complex. The IL-6 family of cytokines shares the gp130 receptor subunit in the formation of their respective heteromeric receptors (Taga and Kishimoto 1997). A recently described cytokine, named IL-31, has been classified by Dillon *et al* as a novel member of the gp130-IL6 family, because its receptor is a heterodimer comprising gp130-like type I cytokine receptor (GPL) and an OSMR subunit (Dillon, Sprecher et al. 2004).

Different publications have reported that some members of the IL-6 family may be implicated in certain skin diseases and wound healing processes. IL-6, IL-11, LIF and OSM have been found to be increased in psoriatic lesions (Bonifati, Mussi et al. 1998), and IL-6 and LIF are produced by purified keratinocytes (Paglia, Kondo et al. 1996; Sugawara, Gallucci et al. 2001). An impaired wound healing process has been reported in IL-6 and STAT3 deficient mice (Sano, Itami et al. 1999; Gallucci, Simeonova et al. 2000). However, further studies on cultured keratinocytes isolated from IL-6 deficient mice showed that the action of IL-6 on keratinocyte migration is mediated by dermal fibroblasts. Indeed, IL-6 alone did not significantly modulate the proliferation or migration of said IL-6-deficient keratinocytes, whereas IL-6 significantly induced their migration when co-cultured with dermal fibroblasts (Gallucci, Sloan et al. 2004).

OSM is secreted from activated T cells, monocytes stimulated by cytokines and from dendritic cells. OSM is a pro-inflammatory mediator, which strongly triggers protein synthesis in hepatocytes (Benigni, Fantuzzi et al. 1996). In humans, OSM and LIF display overlapping biological functions in a number of tissues by increasing growth regulation, differentiation, gene expression and cell survival. OSM is also known to elicit some unique biological functions, not shared with LIF, such as growth inhibition of some tumor cell lines or stimulation of AIDS-associated Kaposi's sarcoma cells. These shared and specific functions of OSM are explained by the existence of two types of OSM receptor complexes. Beside the common LIF/OSM receptor complex made of gp130/LIFRβ subunits, OSM is also able to specifically recognize a type II receptor associating gp130 with OSMRβ (also referred to as "OSMR" or "OSM-R"), which is expressed by endothelial cells, hepatic cells, lung cells, fibroblasts, hematopoietic cells and by some tumor cell lines. The subsequent signaling cascade involves activation of the Janus kinase (JAK 1, JAK 2, Tyk 2), followed by an activation of the Signal Transducer and Activator of Transcription (STAT1, STAT3) and of the Map kinase pathways.

In addition to its anti-neoplastic activity and its role in the pro-inflammatory response (Wahl and Wallace 2001); Shoyab et al, US 5,451,506; Richards et al., US 5,744,442), OSM has been described as stimulating the growth of dermal fibroblasts via a MAP kinase-dependent pathway, thereby promoting dermal wound healing (Ihn and Tamaki 2000).

Other cytokines are also known to have an effect on dermis. For example, Dillon *et al* (*supra*) suggest that overexpression of IL-31 may be involved in promoting the dermatitis and epithelial responses that characterize allergic and non-allergic diseases. These authors do not suggest to use IL-31 for promoting skin repair.

Presently, there exists a real need for novel dermatological approaches for improving epidermal repair, especially in the context of inflammation diseases. Inhibiting STAT3 and/or pro-inflammatory cytokines would clearly ameliorate the status of patients suffering from a variety of inflammatory diseases such as psoriasis, atopic dermatitis, professional dermatitis, seborrheic dermatitis, rosacea, erythema, eczema and certain kinds of acne. Acting on the keratinocytes' differentiation and migration is also necessary for treating other specific diseases such as eschars, keratosis, squama, ulcers, ichtyosis, malum perforan pedis and bullous epidermolysis. The phrase "bullous emolysis" designates a number of dermatitis of different origins (such as bleds, burns, autoimmune diseases, ...), leading to a detachment of the epidermis and liquid accumulation between dermis and epidermis. A particular example of bullous emolysis is bullous phemphigoid.

Improving epidermal repair is also important in the cosmetic field, where no efficient compositions exist for improving the aspect of scars, originating either from recent small wounds or from old cuts, spots, stretch marks and the like.

In this context, the inventors found that several cytokines, in particular OSM and IL-31, can enhance the expression of a number of genes involved in inflammatory processes. As described above, these two cytokines bind to different heteromeric receptors, that both comprise OSMRβ as a subunit. The inventors have shown that normal human epidermal keratinocytes express gp130, GPL and OSMRβ.

As disclosed in the experimental examples below, OSM recruits the STAT3 signaling pathways, as well as the MAP kinase pathways in human epidermal keratinocytes. OSM up-regulates the expression of pro-inflammatory genes in these cells, including chemokines, defensin and the psoriasin. OSM also increases the thickness of reconstituted human epidermis and down-regulates a set of differentiation antigens. The inventors have obtained a psoriasis-like phenotype in mice in which intradermal injections of OSM were performed. Interestingly, other cytokines, especially IL-17 and TNFα, act synergistically with OSM and potentiate its effects.

Experiments conducted by the inventors also revealed that IL-31 can mediate keratinocyte migration. The inventors however observed, in glioblastoma and melanoma tumor cells, that the action of IL-31 depends on the type of GPL subunit involved with OSMRβ in the formation of the heteromeric receptor. In particular, they noticed that a short form of GPL receptor exerts a profound inhibitory effect on the signaling of IL-31 and behaves as a dominant negative receptor.

Taken together, these results indicate that OSM and IL-31 play important roles in skin inflammatory processes. These effects require the signal transduction involving the OSMRβ subunit in heteromeric receptors.

A first object of the present invention is hence the use of at least one molecule selected from antagonists and expression inhibitors of OSM acting as inhibitor of a heteromeric receptor having OSMRβ as a subunit, for the preparation of a composition for improving epidermal repair.

As used herein, the term "inhibitor" is defined as any molecule able to inhibit the signal transduction involving the OSMRβ subunit. Examples of such inhibitors are:
- antagonists of the ligands of the heteromeric receptor having OSMRβ as a subunit, in particular antagonists of OSM and IL-31;
- antagonists of the potentiators of OSM and IL-31, in particular antagonists of IL-17, TNFα and IFN-γ;
- antagonists of the OSMRβ subunit itself;
- expression inhibitors of said ligands or potentiators, which means expression inhibitors of OSM, IL-17, TNFα, IL-31 and IFN-γ; and
- expression inhibitors of the OSMRβ subunit itself.
Examples of antagonists of OSM, IL-17, TNFα, IL-31 and IFN-γ are antibodies, for example neutralizing monoclonal antibodies (directed either against the cytokines, or against their receptors, thereby preventing signal transduction), as well as modified cytokines and soluble receptors. A number of neutralizing antibodies against human OSM, IL-17 and TNFα have already been described, some of which are commercialized by R&D, Mineapolis, USA (www.RnDsystems.com): MAB295 (clone 17001) against OSM, MAB317 (clone 41809) against IL-17, and MAB210 (clone 1825) and MAB610 (clone 28401) against TNFα. Anti-TNFα soluble receptors and antibodies are already available for therapeutic use (against rheumatoid arthritis), among which Enbrel® (etanercept), Remicade® (infliximab), and Humira® (adalimumab). Neutralizing monoclonal antibodies can also be used as antagonists of the OSMRβ subunit. Alternatively, the inhibitor is a transmembrane receptor that acts as a decoy receptor by "trapping" the ligands of the heteromeric receptors having OSMRβ as a subunit. The short form of the GPL subunit can be used according to this specific embodiment. Mutated cytokines, native or mutated peptides different from a cytokine, antibodies, and non-peptidic synthetic or natural molecules can thus be used as inhibitors according to the invention.

Examples of expression inhibitors of OSM, IL-17, TNFα, IL-31, IFN-γ and the OSMRβ subunit are siRNAs, ribozymes, antisens oligonucleotides and the like. An expression inhibitor according to the invention can be, for example, an anti-OSMRβ siRNA such as an isolated double-stranded RNA molecule which is able to mediate interference of the human OSMRβ mRNA, in particular to inactivate the gene encoding OSMRβ, by transcriptional silencing (of course, the same applies to OSM, IL-17, TNFα, IL-31 and IFN-γ). Alternatively, it is possible to use a DNA which is transcribed into anti-OSMRβ siRNA in mammalian cells. For example, it can be a plasmid comprising a palindromic sequence that will be transcribed in the cell into a single-strand RNA molecule designed so that it forms a short hairpin RNA (shRNA). In the cell, this shRNA will be processed into siRNA (Yu, DeRuiter et al. 2002). A number of protocols are now provided to help the skilled artisan design a sequence for a shRNA. For example, mention can be made of the instruction manuals for the pSilencerTM vectors (Ambion® website : http://www.ambion.com). Of course, the obtained polynucleotide sequence can be comprised in a vector for introducing it into a mammalian cell. The skilled artisan will choose, amongst the wide variety of vectors described in the scientific literature, an appropriate vector, depending on the mode of administration that is contemplated (intradermal, subcutaneous, topic, *etc*.), the expected duration of expression of the polynucleotide (depending on the disease), *etc*. Plasmids and viruses, such as lentiviruses, can be cited as non-limitating examples. All these siRNAs, shRNAs, either nude or included in a vector, are herein considered as "expression inhibitors".

In a preferred embodiment of the invention, an OSM antagonist and an antagonist selected from an IL-17 antagonist and/or a TNFα antagonist is/are used for the preparation of a composition. In another referred embodiment, an OSM expression inhibitor and an inhibitor selected from an IL-17 expression inhibitor and/or a TNFα expression inhibitor and/or an OSMRβ expression inhibitor is/are used for the preparation of a composition.

The compositions obtained according to the invention can be used for preventing, reducing and/or treating keratinocyte-mediated inflammation.

In particular, the present invention pertains to the use of at least one antagonist or expression inhibitor of OSM as described above, for the preparation of a composition for preventing, alleviating or treating a skin inflammation disease, such as, for example, psoriasis, atopic dermatitis, bullous epidermolysis, bullous phemphigoid, lichen, acne, eczema, professional dermatitis, seborrheic dermatitis, rosacea, erythema, keratosis and ichtyosis.

Wound healing often results in unsightly scars. Today, no efficient treatment exists for attenuating hypertrophied scars and cheloids. The only proposed solution is plastic surgery. However, cheloids frequently reappear after excision. In such cases, as well as in diseases comprising a thickening of the epidermis, it is most desirable to control the keratinocyte migration. The present invention hence provides novel applications of inhibitors of a heteromeric receptor having OSMRβ as a subunit (like antagonists and expression inhibitors of OSM as above-described), for preparing compositions that inhibit keratinocyte migration, for example for preventing or reducing epidermal thickening. Such compositions can also be used for slowing down epidermal healing. They can be used on established cheloids or scars, for attenuating them.

According to specific embodiments of the present invention, an OSM antagonist as inhibitor of a heteromeric receptor having OSMRβ as a subunit, is used for the preparation of a composition for preventing and/or attenuating chaps on hands, lips, face or body, or for preventing and/or attenuating stretch marks. Other applications of the compositions obtained according to the invention are the improvement of the aspect and comfort of scars, and/or the improvement of the aspect and comfort of epidermal wounds during their healing.

The compositions prepared according to the invention are preferably formulated for topic administration. They can for example be in the form of a cream, lotion, ointment, or dressing.

The invention also pertains to the use of at least two different molecules selected in the group consisting of antagonists and expression inhibitors of , cytokines selected amongst IL-17 TNFα, IL-31 and IFN-γ, and antagonists and expression inhibitors of the OSMRβ subunit, in addition to an antagonist and expression inhibitor of OSM for the preparation of dermatological and/or cosmetic compositions. These at least two components can be either mixed in the same composition, or provided in a kit of parts.

A cosmetic and/or dermatological composition comprising an antagonist of OSM, for example a neutralizing anti-OSM monoclonal antibody, is also part of the invention. In addition or alternatively, a cosmetic and/or dermatological composition according to the invention can comprise an antagonist of IL-17 and/or an antagonist of TNFα.

According to another embodiment of the invention, illustrated in the examples below, OSM as an activator of a heteromeric receptor comprising an OSMRβ subunit is used for preparing a composition that promotes keratinocyte-mediated inflammation. Such a composition can be used, for example, for modifying the secretion, differentiation and migration capacities of keratinocytes, in order to mimic skin pathologies like psoriasis or atopic dermatitis. This is useful for producing *in vitro* and animal models of skin pathologies.

In particular, a method for preparing an *in vitro* model of psoriasis, comprising a step of adding OSM and further comprising adding IL-17 and/or TNFα to cultured keratinocytes, is part of the invention. For example, this method can be performed by adding OSM and further adding IL-17 and/or TNFα to the culture medium of said keratinocytes, at concentrations preferably ranging 0.1 to 100 ng/ml, more preferably from 1 to 10 ng/ml. Optionally, concentrations ranging 0.01 to 10 ng/ml, preferentially 0.1 to 2 ng/ml can be used for each cytokine, especially when several of them are used

The invention also concerns a method for obtaining an animal model to the exclusion of humans of skin diseases such as psoriasis, comprising a step of administering at least one activator of a heteromeric receptor comprising an OSMRβ subunit, in particular OSM and further administering IL-17 and/or INFα, to said animal. In this method, the activator is preferably administered subcutaneously and/or topically and/or via intradermal injection. Animals that can used for performing this method are preferably mammals, such as rodents (rats, mice, etc.), dogs, pigs, primates, and the like. For example, 1 to 10 µg of murine OSM can be injected intradermally to mice, each day during 2 to 8 days, in order to obtain a murine model of psoriasis. Concentrations corresponding to those indicated above for the *in vitro* induction of a psoriasis-like phenotype can typically be used also for obtaining an animal model of psoriasis.

The models of skin pathologies obtained according to the invention can be used for the screening of molecules. *In vitro* models will be referentially used for large (high throughput) screening, and animal models for confirmation of results obtained *in vitro.* These tools will be advantageously used for identifying molecules exhibiting anti-inflammatory properties at the skin level and, hopefully, new active principles for treating skin inflammation diseases.

The invention is further illustrated by the following figures and examples:

### Figures legends

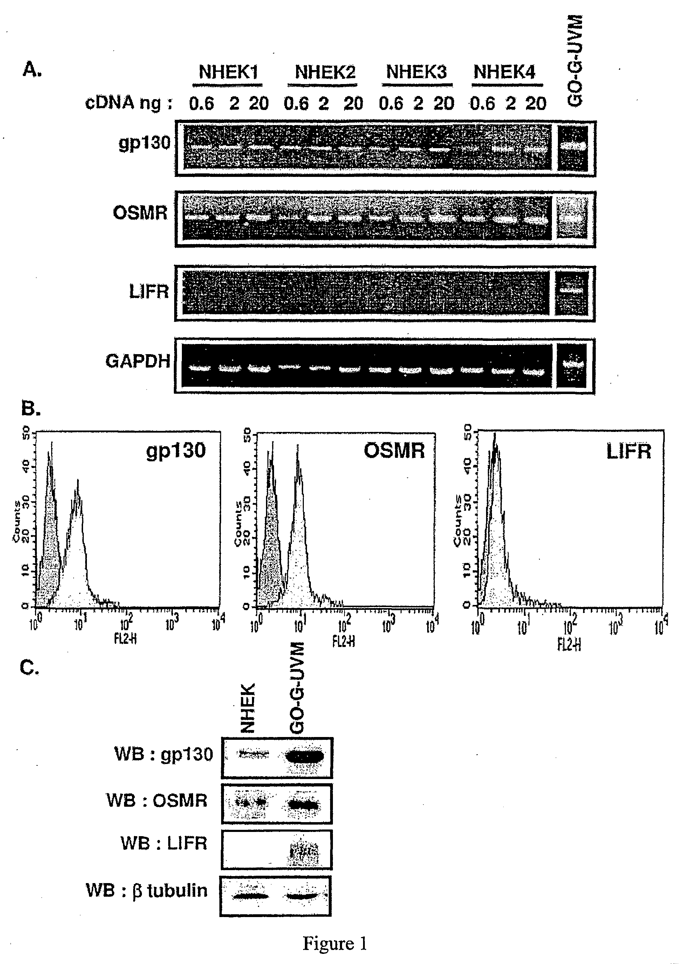

Figure 1 shows expression of OSM receptor by NHEK.
   (A) Total RNA was extracted from NHEK of 4 independent donors. RT-PCR was performed with specific primers for OSMR, gp130, LIFR and GAPDH genes. Serial dilutions of cDNA were amplified to have a semi-quantitative analysis of transcripts expression level. PCR products were analysed by agarose gel electrophoresis. (B) Immunolabelling of cell surface NHEK and flux cytometry analysis. Gp130 and OSMR are clearly detected on the cell, but not the LIFR. (C) Twenty µg of cell lysate from NHEK and the glioblastoma cell line GO-G-UVM were separated by SDS-PAGE (10%) and transferred to nitrocellulose membrane. Ponceau red staining was used to control loading homogeneity. Detection of gp130, OSMR, LIFR and tubulin bands were assessed by Western blot. The results are representative of 3 independent experiments.

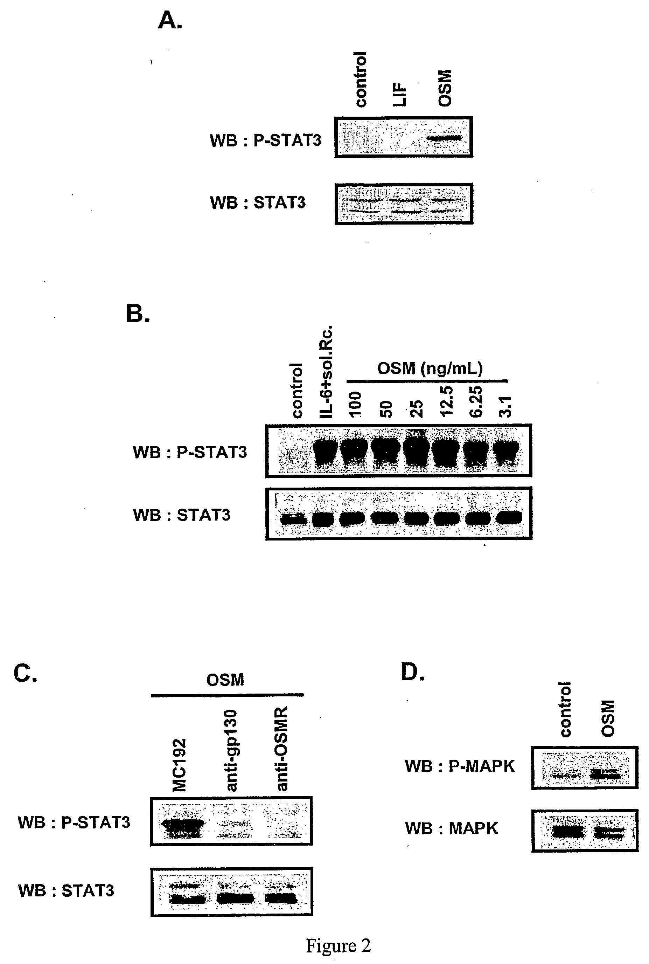

Figure 2 shows induction of STAT3 and MAP kinase phosphorylation by OSM in NHEK.
   (A) NHEK were stimulated or not with LIF or OSM (50 ng/ml). (B) NHEK were stimulated or not for 15 min with 50 ng/ml of IL-5 (negative control) or with 100, 50, 25, 12.5, 6.25 or 3.1 ng/ml of OSM, and phospho-STAT3 (P-STAT3) and STAT3 protein levels were assessed by Western blot. Before stimulation with the cytokines, cells were incubated for 2 h in the presence of neutralizing antibodies, an anti-gp130 (AN-HH1), or an anti-OSMR (XR-M70) monoclonal antibody, or with an isotype control mAb MC192 (final antibody concentration, 15 µg/ml (C). Phospho-MAPK (P-MAPK) and MAPK protein levels in response to OSM was assessed by Western blot (D).

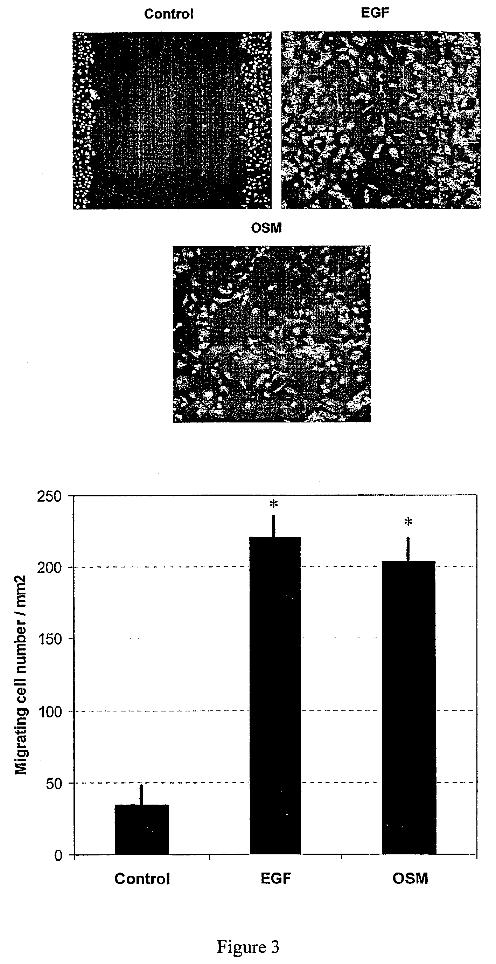

Figure 3 shows effect of OSM on keratinocyte migration.
   *In vitro* wounds were introduced in mitomycin-treated confluent NHEK culture and the keratinocytes were cultured for 48 h with or without 10 ng/ml of EGF or OSM. Cell migration to the cell free area was assessed as described in *Materials and Methods.* Each bar represents the mean ± SEM of migrating keratinocytes counted in 4 non-overlapping fields. One experiment representative of 2.
   * p<0.001 compared with respective control without cytokine, based on Student's *t* test.

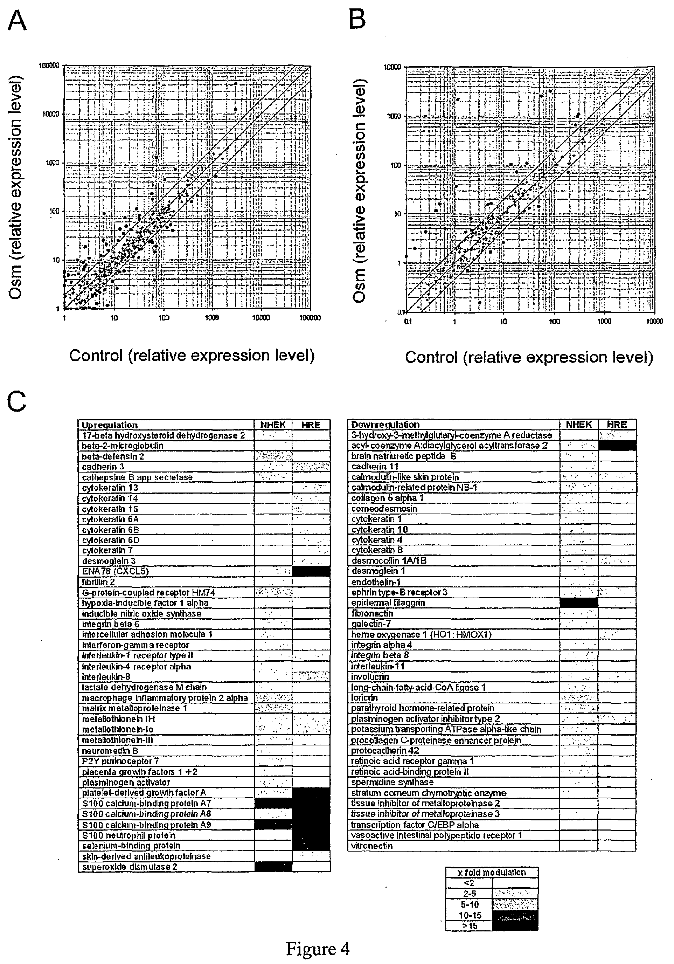

Figure 4 shows expression profiles obtained from OSM stimulated NHEK and OSM treated RHE.
   NHEK (A) or RHE (B) were cultured with or without 10 ng/ml of OSM for 24 h. Total RNA was isolated, treated with Dnase I, and used to make ³³P-labelled cDNA probes, which were hybridized to cDNA arrays. The computer images were obtained after 5 days exposure to a Molecular Dynamics Storm storage screen and further scanning. After local background substraction, an average signal intensity from duplicate spots was normalized for differences in probe labelling using the values obtained for housekeeping genes. (C) The OSM-induced modulation was expressed as the percentage ratio of the signal intensities for cells treated with each cytokine over the signal intensity for unstimulated cells.

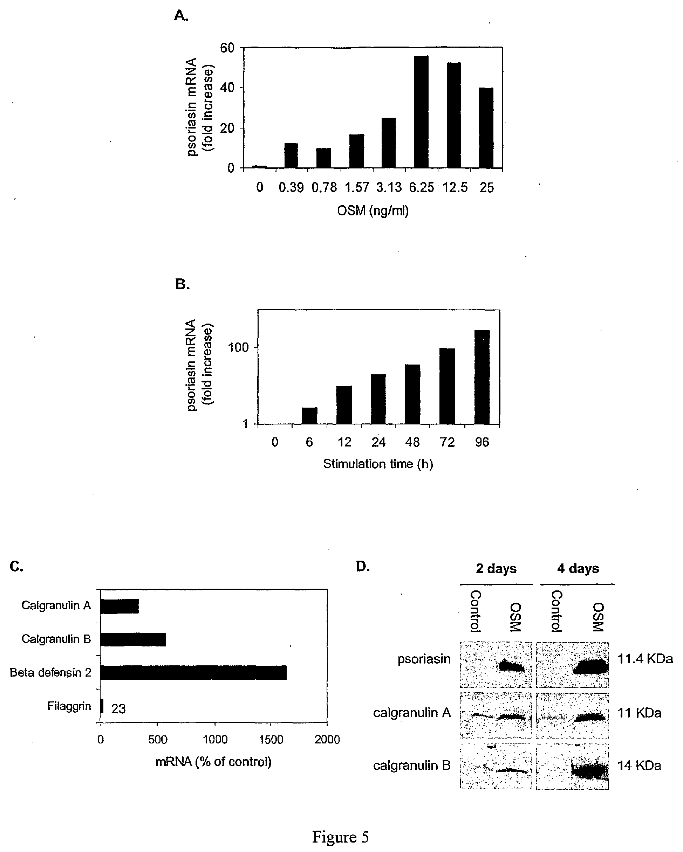

Figure 5 shows effect of OSM on S100A7-9 synthesis by NHEK.
   NHEK were cultured with or without 0.4, 0.8, 1.6, 3.1, 6.3, 12.5 or 25 ng/ml of OSM for 48 h (A) or with or without 10ng/ml of OSM for 6,12, 24, 48, 72, 96 h (B). Total RNA was extracted, reverse transcribed, and S100A7 and HMBS mRNA relative expression was quantified by real time PCR. HMBS was used as a housekeeping gene to normalize gene expression as detailed in *Materials and Methods.* Results, expressed as the relative expression of stimulated cells over control cells, are representative of 2 independent experiments. (C) NHEK were cultured with or without 10 ng/ml of OSM for 48 h. Relative S100A8-calgranulin A, S 100A9-calgranulin B, β-defensin 2 and filaggrin mRNA expression was quantified by quantitative RT-PCR. Results are expressed as the relative expression of stimulated cells over control cells. (D) NHEK were cultured with or without 10 ng/ml of OSM for 48 and 96 h. Twenty µg of cell lysate were separated by SDS-PAGE (16%) and transferred to nitrocellulose membrane. Ponceau red staining was used to control loading homogeneity. S100A7, S100A8 and S100A9 protein levels was determined by Western blot. The results are representative of 3 independent experiments.

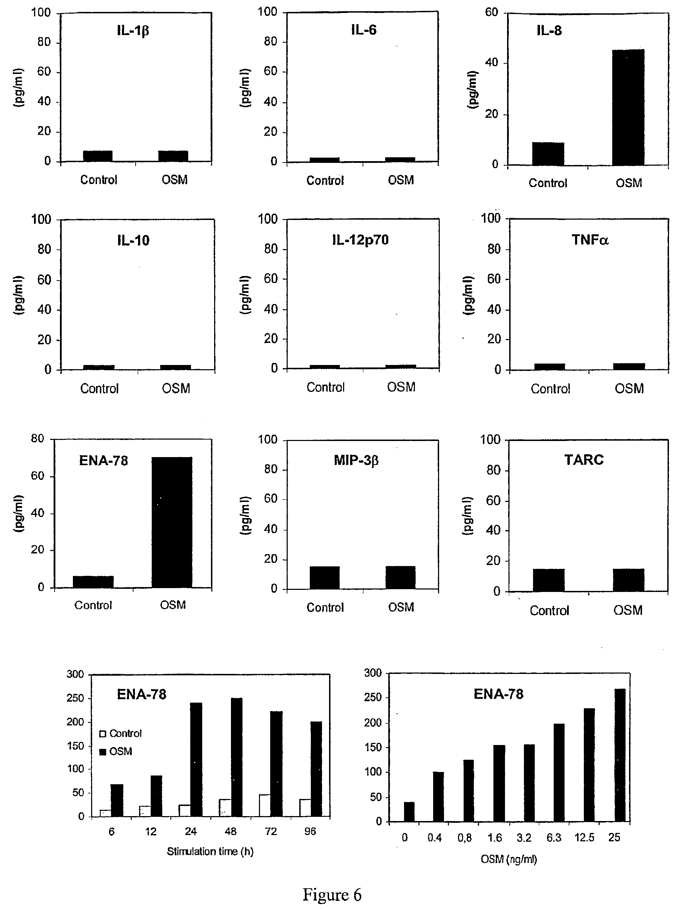

Figure 6 shows cytokines and chemokines production by NHEK. IL-1beta, IL-6, IL-8, IL-10, IL-12p70, TNF alpha, ENA-78, MIP 3 beta were measured by specific ELISA in 48 h NHEK culture supernatants. Cells were cultured in the presence or not of OSM. Dose-response (0.4 to 25 ng/ml) and kinetic (6 to 96h) studies of ENA78 production were also performed.

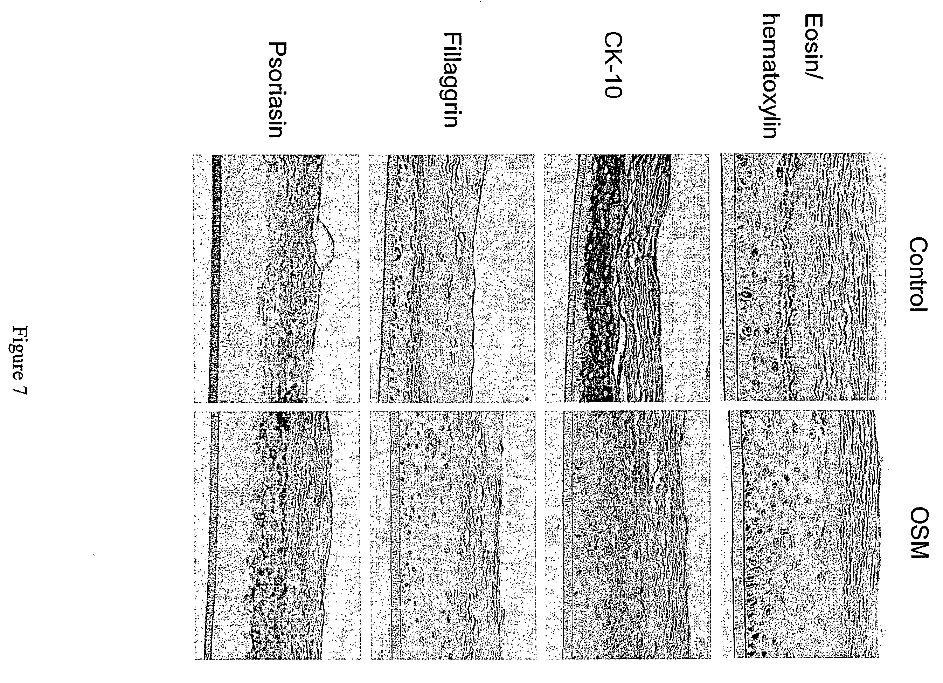

Figure 7 shows histological and immunohistochemical analysis of RHE stimulated or not with 10 ng/ml of OSM for 4 days. RHE were fixed, embedded in paraffin. Four micron vertical sections were stained with hematoxylin/eosin or reacted with anti-K10 keratin mAb, anti-filaggrin mAb or anti-S100A7 mAb and then photographed under a microscope (magnification x 200).

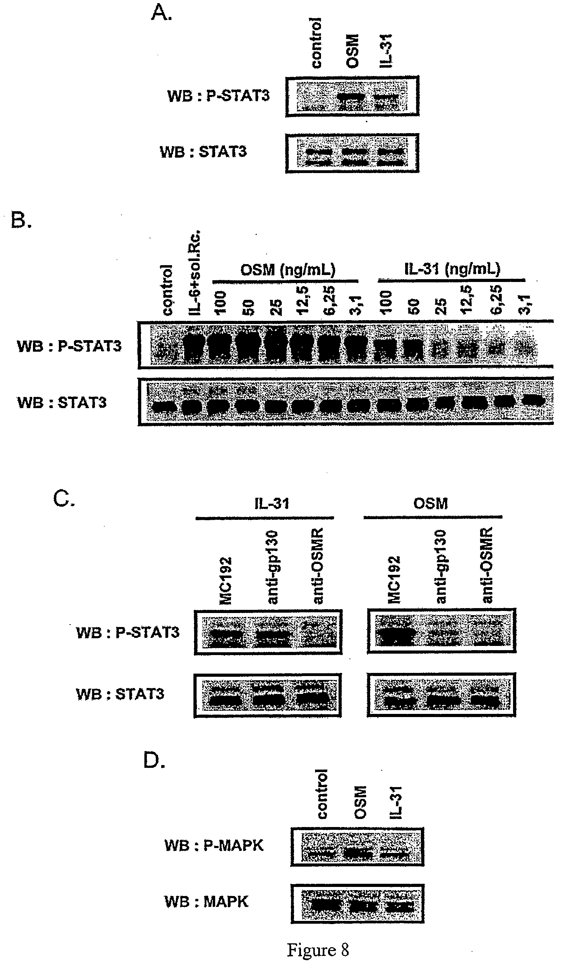

Figure 8 shows induction of STAT3 and MAP kinase phosphorylation by IL-31 and OSM in NHEK.
   NHEK were stimulated or not for 15 min with 10 ng/ml of OSM, or with 100 ng/ml of IL-31 (A), or with different concentrations of these cytokines (B), and phospho-STAT3 (P-STAT3) and STAT3 protein levels were assessed by Western blot. Before stimulation with the cytokines, cells were incubated for 2 h in the presence of neutralizing antibodies, an anti-gp130 (AN-HH1), or an anti-OSMR (XR-M70) monoclonal antibody, or with an isotype control mAb MC192 (final antibody concentration, 15 µg/ml) (C). Phospho-MAPK (P-MAPK) and MAPK protein levels in response to IL-31 and OSM was assessed by Western blot (D).

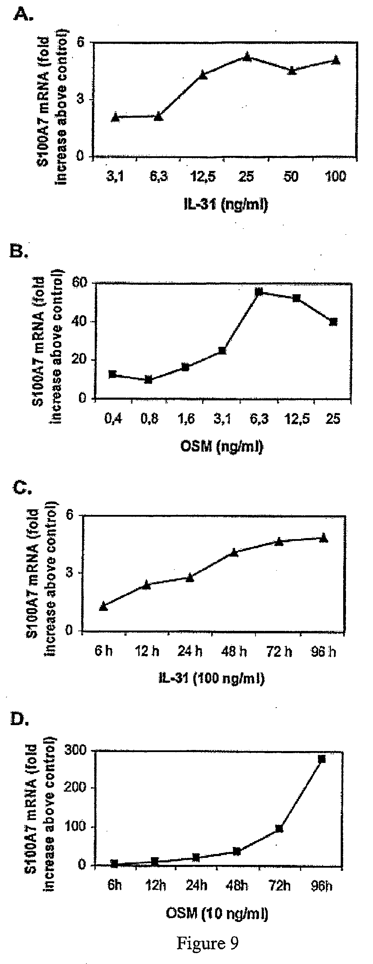

Figure 9 shows effect of IL-31 and OSM on S100A7 mRNA expression.
   NHEK were cultured with or without 3.1, 6.3, 12.5, 25, 50 or 100 ng/ml of IL-31 (A) or with or without 0.4, 0.8, 1.6, 3.1, 6.3, 12.5 or 25 ng/ml of OSM (B) for 48 h. Kinetic study of S100A7 mRNA expression in the absence or presence of 100 ng/ml of IL-31 (C) or 10 ng/ml of OSM (D). Total RNA was extracted, reverse transcribed, and S100A7 and HMBS mRNA relative expression was quantified by real time PCR. HMBS was used as a housekeeping gene to normalize gene expression as detailed in *Material and Methods.* Results, expressed as the relative expression of stimulated cells over control cells, are representative of 2 independent experiments.

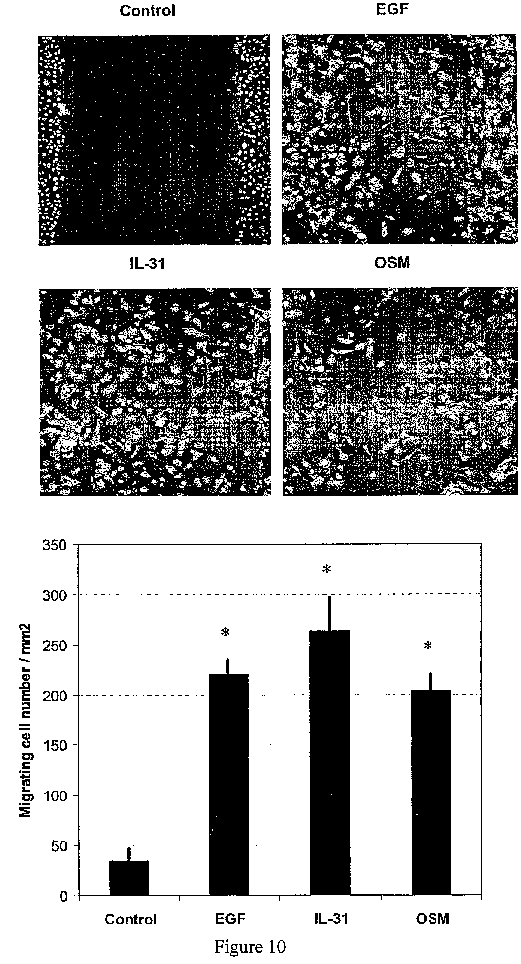

Figure 10 shows effect of IL-31 and OSM on keratinocyte migration.
   *In vitro* wounds were introduced in mitomycin-treated confluent NHEK culture and the keratinocytes were cultured for 48 h with or without 100 ng/ml ofIL-31, or 10 ng/ml of EGF or OSM. Cell migration to the cell free area was assessed as described in *Materials and Methods.* Each bar represents the mean ± SEM of migrating keratinocytes counted in 4 non-overlapping fields. One experiment representative of 2. * p<0.001 compared with respective control without cytokine, based on Student's *t* test.
Figure 11 shows the effect of several cocktails of cytokines on S100A7, hBD4/2, and KRT10 mRNA expression.
   Confluent normal human keratinocytes (NHEK) were treated for 24 hrs with the indicated mixed cytokine (each cytokine at 1 ng/ml final concentration). Total RNA was extracted, reverse-transcribed and the expression of the selected genes was analyzed by real-time PCR.
Figure 12 shows OSM and type II OSM receptor expression in psoriasis
   (A). Ten µm cryostat sections of skin biopsy from healthy donors or psoriatic patients were fixed and subjected to immunohistochemistry for OSM, gp130, OSMRβ, LIFRβ S100A7 and filaggrin. One experiment representative of 4 (magnification x 100).
   (B). OSM transcripts were PCR-quantified from total RNA extracted from healthy or psoriatic lesional skins.
   (C). Lesion infiltrating T cells or peripheral blood circulating T cells were *in vitro* expanded by one stimulation using anti-CD3, anti-CD28 mAbs and IL-2. After a 12-days culture period, cells were stimulated again under the same conditions and 24 h culture supernatants harvested for OSM ELISA determination. p-values were calculated using Student's *t* test.
   (D). The co-localization of OSM and CD3 expression in lesional skin infiltrating T cells was analyzed by double immunofluorescent staining. Dotted lines indicate the border between epidermis (top) and dermis (bottom).

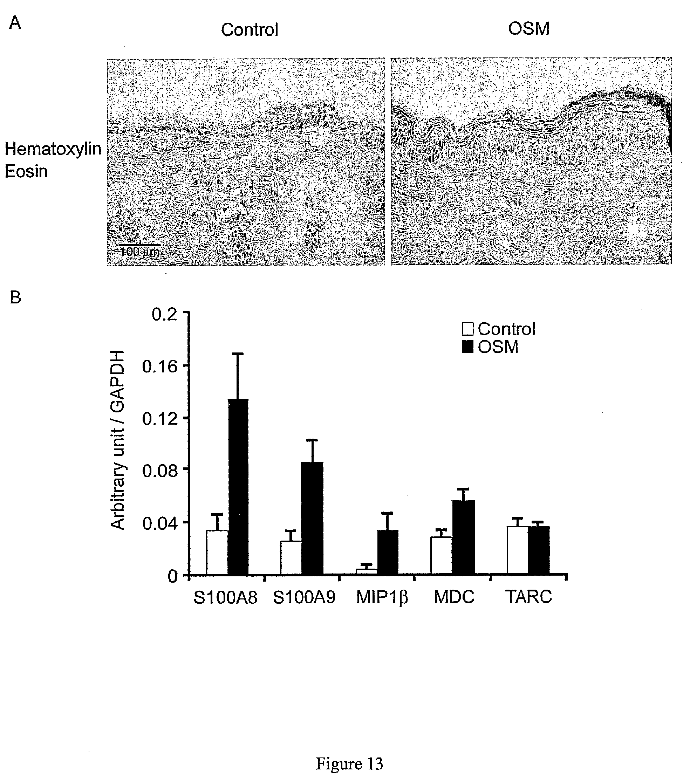

Figure 13 shows the result of intradermal injection of OSM in 12956/SvEv mice
   Four mice were treated with 5 µg of OSM or vehicle alone for 4 days.
   (A). Ten µm cryostat sections of skin biopsies were counterstained with hematoxylin and eosin and photographed under a microscope (magnification x 400). One mouse representative of 4.
   (B). Quantitative RT-PCR analysis of S100A8, S100A9, MIP-1β, MDC and TARC mRNA expression in skin sample from control or OSM-treated mice (mean ± SEM of 4 independent mice).

### EXAMPLE 1: Material and Methods

### Cell culture

NHEK were obtained from surgical samples of healthy breast skin. The use of these samples for research studies was approved by the Ethical Committee of the Poitiers Hospital. Skin samples were incubated overnight at 4°C in a dispase solution (25 U/ml ; Invitrogen Life Technologies, Cergy Pontoise, France). Epidermal sheets were removed from dermis and NHEK were dissociated by trypsin digestion (trypsin-EDTA, Invitrogen) for 15 min at 37°C. Cells were cultured in Serum-Free Keratinocyte Medium (Keratinocyte SFM) (Invitrogen, Cergy Pontoise, France) supplemented with bovine pituitary extract (25 µg/ml) and recombinant epidermal growth factor (0.25 ng/ml ; all purchased from Invitrogen). NHEK were starved for 48 h in Keratinocyte SFM without addition of growth factors before stimulation.

RHE were purchased from SkinEthic Laboratories (Nice, France). They consist of a multi-layered epidermis grown for 12 days at the air-medium interface and which exhibits morphological and growth characteristics similar to human skin (Rosdy et al., 1997). Skin-infiltrating or peripheral blood T cells were expanded using magnetic beads coated with anti-CD3 (SPV-T3b) and anti-CD28 (L293) mAbs (Expander beads^{®}: Dynal, Oslo, Norway) as described previously(Yssel, Lecart et al. 2001; Trickett and Kwan 2003).

Skin-infiltrating T cells were generated from 3 mm punch biopsies from active psoriatic lesions that were extensively washed and transferred to a well of a 24-well tissue culture plate (Nunc, Roskilde, Denmark), in the presence of 5 µl of Expander beads^{®} in RPMI medium, supplemented with 10% Fetal Calf Serum and 10 ng/ml IL-2 in a final volume of 1 ml and incubated at 37°C at 5% CO₂. Peripheral blood mononuclear cells were isolated via Ficoll Hypaque density centrifugation and 10⁵ lymphocytes were cultured in the presence of Expander beads^{®}, as described above. After 3 days of culture, fresh culture medium, containing 10 ng/ml IL-2 was added to the cultures and growing T cells were collected after 10-14 days of culture for use in subsequent experiments. In order to preserve the functional and phenotypic properties of the skin-infiltrating and peripheral blood T lymphocytes, cells were stimulated with Expander beads^{®} only once and not restimulated for further propagation. Results from immunofluorescence and flow cytometric analysis showed that T cells expanded from each of psoriatic skin lesions were 60%-80% CD8⁺ (data not shown). This typical CD4/CD8 ratio was not due to the preferential outgrowth of CD8⁺ T cells under these experimental conditions. For analysis of cytokine production, 10.10⁶ T cells were activated with 10 µg/ml of immobilized anti-CD3 mAb and 2 µg/ml of anti-CD28 mAb in the presence of 10 ng/ml of IL-2 for 24 h and cytokine production was analyzed by ELISA.

### Cytokines and reagents

Cytokines were purchased from R&D Systems (Oxon, UK) and IL-31 was produced in the laboratory as described previously (Diveu, Lak-Hal et al. 2004). The IgG1 isotype control (MC192), anti-gp130 (AN-HH1, AN-HH2, AN-G30), anti-OSMR antibody (AN-A2), anti-OSMRβ (AN-N2, AN-R2, AN-V2), anti-GPL (AN-GL1, AN-GL3) and anti-LIFRβ (AN-E1) mAbs were produced in the laboratory, the neutralizing anti-OSMRβ Ab (XR-M70) was obtained from Immunex (Seattle, WA) and the anti-OSM mAb (17001) from R&D Systems. Polyclonal anti-gp130, anti-LIFRβ, anti-STAT3, anti-S100A8-calgranulin A and anti-S100A9-calgranulin B Abs were from Santa Cruz Biotechnology (Santa Cruz, CA, USA). Anti-S100A7-psoriasin and anti-β-tubulin Abs were purchased from Imgenex (San Diego, CA, USA) and Sigma (St. Louis, Mo) respectively. Antibodies raised against phospho-STAT3, phospho-MAPK and MAPK were from Upstate Biotechnology (Lake Placid, NY). Fluorescein isothiocyanate (FITC)-conjugated anti-CD3 and phycoerythrin (PE)-conjugated anti-CD4 and anti-CD8 mAbs were purchased from BD Biosciences. Antibodies against CK-10 and filaggrin were from Lab Vision Corporation (Fremont, CA, USA). Goat anti-mouse and anti-rabbit peroxidase-labeled immunoglobulins were from Clinisciences (Montrouge, France), rabbit anti-goat peroxidase-conjugated Ab was from Sigma, and streptavidin-Alexa Fluor 568 conjugated from Invitrogen. IL-1β, IL-6, IL-8, IL-10, IL-12p70 and TNFα were quantified using BD^{™} Cytometric Bead Array (CBA) Human Inflammation kit (BD Biosciences, Mountain View, CA, USA). Detection of OSM, ENA-78, MIP-3β, TARC and VEGF was carried out using ELISA kits purchased from R&D Systems.

### RT-PCR and RT-real time PCR analysis

Total cellular RNA was isolated using Trizol reagent (Invitrogen) and treated with DNase I (0.05 U/µl ; Clontech, Palo Alto, CA, USA). cDNAs were synthesised from 2 µg of total RNA by random hexamer primers using MMLV reverse transcriptase (Promega, Madison, WI). Reverse transcription products were subsequently amplified by 25 cycles of PCR using primers for OSMR (forward 5'-CCTGCCTACCTGAAAACCAG-3' (SEQ ID No: 1) and reverse 5'-ACATTGGTGCCTTCTTCCAC-3' (SEQ ID No: 2)), gp130 (forward 5'-GGGCAATATGACTCTTTGAAGG-3' (SEQ ID No: 3) and reverse 5'-TTCCTGTTGATGTTCAGAATGG-3' (SEQ ID No: 4)), LIFR (forward 5'-CAGTACAAGAGCAGCGGAAT-3' (SEQ ID No: 5) and reverse 5'-CCAGTCCATAAGGCATGGTT-3' (SEQ ID No: 6)) and GAPDH (forward 5'-ACCACAGTCCATGCCATCAC-3' (SEQ ID No: 7) and reverse TCCACCACCCTGTTGCTGTA (SEQ ID No: 8)). Amplified products were analysed by 2% agarose gel electrophoresis.

Quantitative real time PCR was carried out using the LightCycler-FastStart DNA Master^{PLUS} SYBR Green I kit (Roche, Mannheim, Germany). The reaction components were 1X FastStart DNA Master^{PLUS} SYBR Green I and 0.5 µM of forward and reverse primers for S100A7 (forward 5'-GCATGATCGACATGTTTCACAAATACAC-3' (SEQ ID No: 9) and reverse 5'-TGGTAGTCTGTGGCTATGTCTCCC-3' (SEQ ID No: 10)), S100A8 (Pattyn, Speleman et al. 2003), S100A9 (forward 5'-GCTCCTCGGCTTTGACAGAGTGCAAG-3' (SEQ ID No: 11) and reverse 5'-GCATTTGTGTCCAGGTCCTCCATGATGTGT-3' (SEQ ID No: 12)), hBD2/4 (forward 5'-GCCATCAGCCATGAGGGTCTTG-3' (SEQ ID No: 13) and reverse 5'-AATCCGCATCAGCCACAGCAG-3' (SEQ ID No: 14)), KRT10 (forward 5'-GCCCGACGGTAGAGTTCTTT-3' (SEQ ID No: 15) and reverse 5'-CAGAAACCACAAAACACCTTG-3' (SEQ ID No: 16)), OSM (forward 5'-TCAGTCTGGTCCTTGCACTC-3' (SEQ ID No: 17) and reverse 5'-CTGCAGTGCTCTCTCAGTTT-3' (SEQ ID No: 18)), and GAPDH (forward 5'-GAAGGTGAAGGTCGGAGTC-3' (SEQ ID No: 19) and reverse 5'-GAAGATGGTGATGGGATTTC-3' (SEQ ID No: 20)) and hydroxymethyl-bilane synthase HMBS) (Vandesompele, De Preter et al. 2002) as housekeeping genes. After cDNA fluorescent quantification using propidium iodide, 250 ng, 25 ng and 2.5 ng of cDNA were added as PCR template in the LightCycler glass capillaries. The cycling conditions comprised a 10 min polymerase activation at 95°C and 50 cycles at 95°C for 10 s, 64°C for 5 s and 72°C for 18 s with a single fluorescence measurement. Melting curve analysis, obtained by increasing temperature from 60°C to 95°C with a heating rate of 0.1 °C per second and a continuous fluorescence measurement, revealed a single narrow peak of suspected fusion temperature. A mathematical model was used to determine the relative quantification of target genes compared to HMBS reference gene (Pfaffl 2001).

For *in vivo* studies, RNA from control or skin site injected with OSM were extracted, reverse-transcribed and used to perform quantitative RT-PCR with specific primers for murine S100A8 (forward 5'-TCCAATATACAAGGAAATCACC-3' (SEQ ID No: 21) and reverse 5'-TTTATCACCATCGCAAGG-3' (SEQ ID No: 22)), murine S100A9 (forward 5'- GAAGGAATTCAGACAAATGG-3' (SEQ ID No: 23) and reverse 5'-ATCAACTTTGCCATCAGC-3' (SEQ ID No: 24)), murine MIP-1β (forward 5'-CCTCTCTCTCCTCTTGCTC-3' (SEQ ID No: 25) and reverse 5'-AGATCTGTCTGCCTCTTTTG-3' (SEQ ID No: 26)), murine MDC (forward 5'-TGCTGCCAGGACTACATC-3' (SEQ ID No: 27) and reverse 5'-TAGCTTCTTCACCCAGACC-3' (SEQ ID No: 28)), murine TARC (forward 5'-CATTCCTATCAGGAAGTTGG-3' (SEQ ID No: 29) and reverse 5'-CTTGGGTTTTTCACCAATC-3' (SEQ ID No: 30)) and murine GAPDH (forward 5'-ATCAAGAAGGTGGTGAAGC-3' (SEQ ID No: 31) and reverse 5'-GCCGTATTCATTGTCATACC-3' (SEQ ID No: 32)) as housekeeping gene.

### Gene expression profiling using cDNA macroarrays

Total RNA was isolated as described for PCR studies. DNase treatment, polyA⁺ RNA enrichment, ³³P-labelled cDNA probe synthesis, purification and hybridization to custom Atlas array membranes (Bernard, Pedretti et al. 2002) were performed according to Clontech's recommendations (Clontech, Palo Alto, USA). Membranes were exposed for 5 days to a Molecular Dynamics Storm storage screen and scanned using a phosphorimager scanner (Molecular Dynamics Storm analyser, Amersham Biosciences, Uppsala, Sweden). After local background substraction, average signal intensity from duplicate spots was normalized for differences in probe labelling using the values obtained for housekeeping genes (Bernard et al., 2002). For each gene, the OSM-induced modulation was expressed as the relative expression value for stimulated versus control sample. Arbitrarily, only modulation above 2 was considered significant for confirmation using RT-real time PCR assay.

### Western blotting analysis

For STAT3 and MAPK phosphorylation, NHEK were stimulated for 15 min in the presence of the indicated cytokine. Cells were lysed in SDS sample buffer (62.5 mM Tris-HCl pH 6.8, 2% SDS, 10% glycerol, 50mM DTT, 0.1% bromophenol blue), sonicated and then submitted to SDS-PAGE and transferred onto an Immobilon membrane. The membranes were subsequently incubated overnight with the primary antibody, before being incubated with the appropriate peroxidase-labelled secondary antibody for 60 min. The reaction was visualized by chemiluminescence according to the manufacturer's instructions. Membranes were stripped in 0.1 M glycine pH 2.8 for 2 h and neutralized in 1 M Tris-HCl pH 7.6 before reblotting. For neutralizing experiments, NHEK were incubated with the appropriate antibodies for 2 h before stimulation.

To determine the expression of gp130, LIFR and OSMR, the cells were lysed in 10 mM Tris HCl pH 7.6, 5 mM EDTA, 50 mM NaCl, 30 mM sodium pyrophosphate, 50 mM sodium fluoride, 1 mM sodium orthovanadate proteinase inhibitor and 1% Brij 96. After lysis and centrifugation to remove cellular debris, the supernatants were then treated as described above.

For S100 proteins expression, NHEK were stimulated for 2 days in the presence of OSM (10 ng/ml). Cell lysis was performed with 50 mM Tris HCl pH 7.4, 150 mM NaCl, 1 mM EDTA, 1% Triton, 1% sodium deoxycholate, 0,1 % SDS, 1 mM PMSF, 1 mM sodium orthovanadate, 1% proteases inhibitors. S100A7, S100A8 and S100A9 were detected by immunochemistry as described above. Ponceau red staining was used to control loading homogeneity.

### In vitro keratinocyte migration assay

Keratinocytes were cultured in wells pre-coated with type I collagen (200 µg/ml, Institut Jacques Boy, Reims, France) until they reached 80 % confluency. Cells were starved for 48 h in Keratinocyte SFM and then treated with 10 µg/ml of mitomycin C (Sigma) for 2 h to prevent cell proliferation. A cell-free area was created by scraping the keratinocyte monolayer with a plastic pipette tip. Keratinocytes migration to the cell-free area was evaluated after 48 h of culture in the absence or presence of EGF or OSM. Using an inverted phase contrast microscope. The number of migrating keratinocytes was counted in 4 non-overlapping fields. Values represent the mean ± SEM of cells per mm² beyond the frontiers of the *in vitro* injury. Student's t test was used for statistical analysis.

### Reconstituted human epidermis model

For histological and immunohistochemical studies, RHE, grown for 12 days at the air-medium interface, were purchased from SkinEthic Laboratories (Nice, France). They consist of a multi-layered epidermis which exhibit morphological and growth characteristics similar to human skin (Rosdy, Bertino et al. 1997). As recommended, RHE were grown for 1 day in SkinEthic growth medium prior to stimulation in the absence or presence of OSM for 4 days. They were then fixed in a balanced 10% formalin solution and embedded in paraffin. Four micron vertical sections were stained with heamatoxylin/eosin or with specific Ab and peroxidase-conjugated Antibodies, and counterstained with haematoxylin according to standard protocols (Rosdy, Bertino et al. 1997). Anti-K10 keratin and anti-filaggrin monoclonal Antibodies were from Lab Vision Corporation (Fremont, CA, USA).

For gene expression profiling using cDNA macroarrays, 17 days old RHE were grown for 1 day in SkinEthic maintenance medium prior to stimulation in the absence or presence of OSM for 24 h. Total RNA was isolated and cDNA arrays were performed as described above.

### Immunofluorescence and immunohistochemistry analyses

Cells were incubated for 30 min at 4°C with the appropriate primary Ab (AN-G30, AN-N2, AN-E1) or with an isotype control Ab followed by a PE-conjugated anti-mouse Ab incubation. Fluorescence was analysed on a FACSCalibur® flow cytometer equipped with Cellquest software (Becton-Dickinson). For immunohistochemistry, 10 µm cryostat sections were fixed, permeabilized and immunostained with the relevant primary Ab (AN-HH2, AN-R2, AN-GL1, AN-E1, 17001) and the avidin peroxidase method (Vector Laboratories, Burlingame, CA, USA). Sections were counterstained with Mayer's hematoxylin (Sigma) before mounting. For immunofluorescent staining, a biotin-labeled anti-OSM mAb was used and detected with streptavidin- Alexa Fluor 568 conjugated together with a FITC-conjugated anti-CD3 mAb. Nuclei were counterstained with To-Pro 3 (Invitrogen). Immunostainings were analyzed with the Olympus FV1000 confocal microscope. Twelve-day grown RHE were cultured for 4 additional days in the presence or absence of OSM or IL-31. Cultures were then fixed with a formalin solution and embedded in paraffin. Four µm vertical sections were stained according to standard protocols.

### Murine model

All animal experiments were approved by the Institutional Animal Care Committee. Hair was removed from the back of 129S6/SvEv 9-week old mice. Three days later, mice were injected intradermally with 5 µg of murine OSM or vehicle control in two locations on either side of the back. Injections were carried out daily during 4 days until sacrifice. Mice were euthanized using carbon dioxide. Skin samples were removed from the prepared area and were embedded and frozen in Tissue Tek OCT compound for histological analysis or directly frozen in liquid nitrogen for mRNA extraction. Ten µm sections were fixed and counterstained with Mayer's hematoxylin and eosin (Sigma).

### EXAMPLE 2: Human keratinocytes express the type OSM receptor on their surface

To show the potential functions of OSM in normal human keratinocytes the inventors first undertook an analysis of its receptor chain expression. To determine the nature of expressed type I or type II receptors, RT-PCR for gp130, LIFRβ and OSMRβ were carried out starting from primary cultures of keratinocytes. CO-G-UVM and glioblastoma cells were used as controls for LIFR. Obtained results show that NHEK predominantly expressed transcripts for OSMR and the gp130, whereas only low levels of the LIFR chain could be evidenced (Fig. 1A). The RNA analysis was further reinforced by measuring the expression levels of corresponding proteins by flow cytometry. Fluorescence analyses revealed a clear expression of gp130 and OSMRβ on the NHEK cell surface (Fig 1B). In contrast no detection of membrane LIFRβ expression could be evidenced, whereas the anti-LIFRβ antibody gave the expected result when incubated with a cell line used as a positive control. This was further supported by western blot analyses showing the detection of gp130 and OSMRβ chains, and an absence of LIFRβ expression in NHEK (Fig1C). Similar experiments carried out on samples coming from four different donors led to the same results, ruling out the possibility for variations in type I or type II OSM receptor expression from donor to donor. These first results indicate that human keratinocytes preferentially expressed the specific type II OSM receptor.

### EXAMPLE 3 : STAT-3 and MAP kinase pathways are recruited by OSM in human keratinocytes.

The inventors show OSM-induced signal transduction in NHEK. Since STAT3 is usually recruited by the OSM type II receptor pathway, they analyzed the tyrosine phosphorylation of the signaling molecule in response to increasing concentrations of the cytokine. A strong induction of tyrosine phosphorylation was observed for STAT3, with plateau level values still present down to 3 ng/ml OSM (Fig. 2B). The involvment of gp130 and OSMRβ subunits was further demonstrated by blocking of the STAT3 phosphorylation when adding receptor neutralizing mAbs to the NHEK culture before OSM contact (Fig. 2C). Importantly, the complete neutralization of STAT3 phosphorylation observed in the presence of the anti-OSMRβ mAb further demonstrated the absence of recruitment by OSM of the share LIF/OSM type I receptor in NHEK. In agreement with this observation, after a LIF contact no evidence for a STAT3 activation could be observed in NHEK.

In addition, type II OSM receptor complex is also known to be a more potent activator for the recruitment of the Map kinase pathway compared to the shared LIF/OSM receptor. A cooperative effect between ERK1/ERK2 and the Shc adaptor, and mediated through OSMRβ, but not through the LIFRb, explains this strong activation the MAP kinase pathway in response to OSM (Boulton, Stahl et al. 1994). The ERK1/2 signaling in response to the cytokine in NHEK was therefore analyzed by determining their tyrosine phosphorylation level. As expected, NHEK stimulation with OSM quickly increased the MAP kinase phosphorylation (Fig.2D). Taken together, these results demonstrated that gp130/OSMRβ receptor complex expressed in human keratinocytes is fully functional, and that the entire observed signals are mediated through the type II receptor.

### EXAMPLE 4 : OSM is a potent inducer of keratinocyte migration.

To underline the functional responses of NEHK to OSM, The inventors analyzed the potential effect of OSM on an *in vitro* model mimicking the wound healing and based on the keratinocyte migration (Kira, Sano et al. 2002). Forty eight hours after initiation of the culture, cells present in the middle of the well were removed by scratching, and the remaining keratinocytes were stimulated with either EGF, known to trigger the keratinocyte migration, or with OSM. After an additional 36h of culture, the cytokine potential for inducing cell migration was visually determined or by cell counting (Figure 3). Obtained results show that OSM led to an important migration of NHEK, similar to that observed in the presence of EGF.

### EXAMPLE 5 : Identification of OSM-induced gene expression in human keratinocytes

To have a better view of the NHEK functional response the inventors analyzed the modification of keratinocyte gene expression profile induced by OSM using cDNA arrays. Used arrays were specially designed for the study of keratinocytes, and consisting of 586 different cDNAs spotted in duplicate. They consisted in genes involved in keratinocyte cell structure, metabolism, extracellular matrix, adhesion, differentiation, signaling, signal transduction, apoptosis and stress (Bernard et al., 2002). RNA extracted from control or OSM-stimulated NHEK were used to generate labeled cDNA probes by reverse transcription. Probing the Atlas cDNA array membranes with these cDNA probes revealed that OSM increased the expression of 36 genes and decreased the expression of 38 genes. OSM down regulates a large set of genes associated with keratinocyte differentiation, such as cytokeratin (CK)1, CK10, fillagrin and loricrin genes. Among the up-regulated genes, the inventors found a marked increase for the calcium binding proteins, psoriasin (S100A7), calgranulins (S100A8, S100A9) and the S100 neutrophil protein (Fig 4). Interestingly, the expression of these proteins is known to be up-regulated in inflammatory tissues (Madsen, Rasmussen et al. 1991; Nagase and Woessner 1999; Roth, Vogl et al. 2003). OSM also induced the G-protein-coupled receptor HM74, the super oxyde dismutase 2 and the beta-defensin genes, involved in tissue protection. Genes involved in tissue remodelling such as matrix metalloproteinase 1 and tenascin were also induced by OSM. In addition, OSM increased the expression of the chemokines CXCL1 (MIP-2a), CXCL5 (epithelial-derived neutrophil-activating peptide (ENA 78) and CXCL8 (IL-8), and the platelet-derived growth factor A (PDGF-A) genes.

Obtained results indicate that, in human keratinocytes, OSM was able to recruit a number of genes involved in inflammatory processes and in innate immune response.

### EXAMPLE 6 : OSM induced keratinocytes to produce psoriasin, calgranulin, β defensin, and chemokines

To further reinforce the results obtained using designed-arrays, quantitative analyses at mRNA and protein levels were carried out for a selected number of identified genes. Quantitative analysis of psoriasin / S100A7 mRNA expression in reponse to OSM was performed by RT-real-time PCR along kinetic and dose-response studies. The inventors show that psoriasin / S100A7 mRNA was up-regulated in a dose-dependent manner in response to OSM ranging from 1.6 to 6.3 ng/ml after a 48-h treatment, and the plateau was reached for 6.3 ng/ml OSM with a fifty fold increase of the signal above control (Fig. 5A). Kinetic study revealed an increase in psoriasin / S100A7 mRNA expression starting at 12 h following stimulation with 10 ng/ml of OSM (Fig. 5B). It still increased up to 96 h, with a strong induction of about 290 folds above the control value. This was confirmed at the protein level by western blot analyses of the psoriasin / S100A7, as well as of two related calcium binding proteins, the S100A8 and S100A 9 calgranulins (Figure 5D). Results show that NHEK exposure to 10 ng/ml of OSM resulted in an increased expression of studied proteins that was strongest at day 4 than at day 2 (Fig. 5D). Figure 5C depicts the results obtained by analyzing the RNA quantitative expression of filagrin and defensin-2, two important markers of skin activation.

The production of the chemokines CXCL5 and CXCL8 in 48h NHEK is also clearly enhanced under OSM stimulation (Fig. 6B).

### EXAMPLE 7 : OSM triggers hyperplasia of reconstituted human epidermis and modulates further expression the dynamic related antigens.

To further approach the dynamic of epidermal differentiation, the inventors tested the biological effect of OSM on *in vitro* RHE in order to assess the basal cell layer proliferation and the graduated epidermal differentiation processes. Histological analysis of control RHE showed a keratinised multi-stratified epithelium resembling epidermis *in vivo,* containing intact basal, spinous, granulous and cornified cell-layers, and numerous keratohyalin granules in the upper granular layer (Fig.7). OSM triggered the hyperplasia of the keratinocytes layers, leading to an increase in the overall thickness of the RHE. In addition, they observed a loss of keratohyalin granules in the granular layer and the presence of picnotic nuclei. cDNA array profile analysis of RHE confirmed that OSM strongly up-regulated S100A7, S100A8, S100A9 and S100 neutrophil protein genes, as previously described on NHEK (Fig.4). By immunohistochemistry, we confirmed the S100A7 protein up-regulation in OSM-treated RHE (Fig. 7). In agreement with the data on NHEK, OSM treatment on RHE also up-regulated CXCL5, CXCL8 chemokine genes, and the PDGF-A and the cadherin 3 gene transcription. Specific to RHE but not detected in NHEK, OSM up-regulated the CK6A, 6B, 6D, 7, 13, 14, 16, the skin-derived antileukoproteinase and the TGFβ-inducible early protein.

On the other hand, OSM down-regulates genes associated with keratinocyte differentiation, such as involucrin, filaggrin, and calmodulin-like skin protein (Mehul, Bernard et al. 2000; Rogers, Kobayashi et al. 2001; Jonak, Klosner et al. 2002; Wagener, van Beurden et al. 2003). Immunohistochemical analysis performed on RHE sections confirmed the inhibition of keratinocyte differentiation, as indicated by the decrease of filaggrin and keratin 10 expression in OSM treated RHE (Fig. 7).

### EXAMPLE 8: Discussion

The use of a cDNA array approach, specially designed for the analysis of gene expression in human skin, enabled the identification of OSM target genes in human keratinocytes and the demonstation of the involvement of OSM in a variety of processes, including migration and differentiation. In particular, the strong, dose dependent, OSM-mediated induction of the expression of S100A7, S100A8 and S100A9 proteins in NHEK and RHE demonstrates the pro-inflammatory and chemotactic effects of the cytokine. The opposing effects of IL-10 and OSM in cutaneous inflammation are underscored by the IL-10-induced down-regulation of S100A8 and S100A9 release by monocytes (Lugering, Kucharzik et al. 1997). S100A7, S100A8 and S 1 00A9 belong to the pleiotropic S100 family of calcium-binding proteins (Roth, Vogl et al. 2003). Although their main functions are as yet unclear, they appear to play prominent inflammatory functions (Watson, Leygue et al. 1998; Donato 1999; Roth, Vogl et al. 2003) and to be involved in the tight regulation of a large number of intra- and extracellular activities such as the dynamic of motility of cytoskeletal components or chemotaxis (Ryckman, Vandal et al. 2003). Interestingly, whereas all three S100A7, S100A8 and S100A9 proteins have been reported to be expressed at low or undetectable levels in normal skin epidermis and non-differentiated cultured keratinocytes, they are highly expressed in abnormally differentiated psoriatic keratinocytes (Broome, Ryan et al. 2003), during wound repair (Thorey, Roth et al. 2001) and in epithelial skin tumors (Watson, Leygue et al. 1998; Gebhardt, Breitenbach et al. 2002; Alowami, Qing et al. 2003). Because of the chemotactic effects of S100A7 on inflammatory cells, in particular neutrophils and CD4⁺ T lymphocytes, it has been suggested that S100A7 may be involved in the genesis of psoriatic lesions (Watson, Leygue et al. 1998). Since S100A7 acts upstream of these mechanisms, the inventors demonstrate that OSM is a key molecule for the induction of S100A7 under pathological conditions, and is involved in the pathological state. The modulation of additional genes by OSM is also in favour of the pro-inflammatory and chemotactic roles of OSM. Indeed, the induction of neutrophil attractant chemokine CXCL5/ENA-78 together with the down-regulation of heme oxygenase 1, which antagonizes inflammation by attenuating adhesive interaction and cellular infiltration in skin, could contribute to the neutrophil influx in skin (Koch, Kunkel et al. 1994; Wagener, van Beurden et al. 2003).

OSM-induced MMP-3 expression is also of interest in the context of inflammatory cutaneous diseases and wound repair. Whereas MMP-3 cannot be detected in normal skin, it is expressed by proliferative keratinocytes of the basal layer after injury (Pilcher, Wang et al. 1999). During progression of many diseases, MMP-3 is involved in epidermis remodeling by removal of extracellular matrix during tissue resorption (Nagase and Woessner 1999; Pilcher, Wang et al. 1999), and mice that lack the MMP-3 gene are deficient in wound repair of the epidermis (Bullard, Lund et al. 1999). Using an *in vitro* wound assay, the inventors demonstrated that keratinocyte migration is strongly increased by OSM stimulation. These data are in agreement with the demonstration that STAT3 deficiency in keratinocytes leads to an impaired migration (Sano, Itami et al. 1999). Under inflammatory conditions, OSM appears to be one essential mediator enhancing keratinocyte migration and wound healing, *via* MMP-3 or S100A8-S100A9 dependent mechanisms. Additional evidence to establish the involvement of OSM in wound healing is the strong induction of PDGF in RHE, a major proliferative and migratory stimulus for connective tissue during the initiation of skin repair processes (Rollman, Jensen et al. 2003).

The inventors also showed that OSM increases the overall thickness of the keratinocyte layer of RHE. This process seems not to be related to basal cell hyperproliferation since Ki67 expression is not induced in response to OSM, but more likely results from an inhibition of terminal keratinocyte differentiation, as shown by the decreased production of filaggrin, loricrin or involucrin. OSM down-regulates the expression of the calmodulin-like skin protein (CLSP) and calmodulin-related protein NB-1, two members of the calmodulin family, directly related to keratinocyte differentiation (Mehul, Bernard et al. 2001; Rogers, Kobayashi et al. 2001). CLSP binds transglutaminase-3, a key enzyme implicated in the formation and assembly of proteins, such as loricrin or involucrin, to form the cornified cell envelop of the epidermis (Mehul, Bernard et al. 2000). The modulating effects of OSM on the keratin expression profile, *i.e.,* keratin 6 over-expression and keratin 10 inhibition, also supports the notion of an inhibition of epidermal differentiation. Keratin 6 is known to be induced under hyperproliferative and/or inflammatory situations, including wound healing, psoriasis, carcinogenesis, or by agents such as retinoic acid that provoke epidermal hyperplasia (Navarro, Casatorres et al. 1995; Komine, Rao et al. 2000). In contrast, keratin 10, normally expressed in terminally differentiating epidermal keratinocytes, is reduced during wound healing (Paramio, Casanova et al. 1999).

### EXAMPLE 9: Similar results obtained with IL-31

Studies presented in examples 3, 4 and 5 have been realized with IL-31 instead of OSM and similar results were obtained: IL-31 recruits STAT3 signaling pathways (fig. 8) in NHEK and induces expression of psoriasin (S100A) and calgranulin A and B (S100A8-9) (fig.9). IL-31 is also able to induce keratinocyte migration (fig 10).

### EXAMPLE 10: gamma Interferon potentiates the action of IL-31 on the signal transduction

When NHEK were preincubated 24h in the presence of 50 g/ml of gamma Interferon (INFγ) before IL-31 stimulation (50 ng/ml), P-STAT3 level were increased 3 to 4 folds when compared to NHEK preincubated in medium alone (same studies as those realized for the fig. 8). This demonstrates that INFγ is a modulator of the action of IL-31 on signal transduction.

### EXAMPLE 11: IL-17 and TNFα potentiate the action of OSM on the expression of several inflammation markers

The combined effect of several cytokines on keratinocytes was then tested by measuring the expression of the keratinocyte inflammation markers psoriasin (S100A7) and defensin beta-2/beta-4 (hBD2/4) mRNAs, in the presence of various cytokines cocktails. The effect of these cocktails was also tested on keratin 10 (KRT10) mRNA, since KRT10 is a differentiation marker associated with tissue healing.

To that aim, confluent normal human keratinocytes (NHEK) were treated for 24 hrs with the indicated mix of cytokines (each cytokine at 1 ng/ml final concentration). Total RNA was extracted, reverse-transcribed and the expression of the selected genes was analyzed by real-time PCR as described.

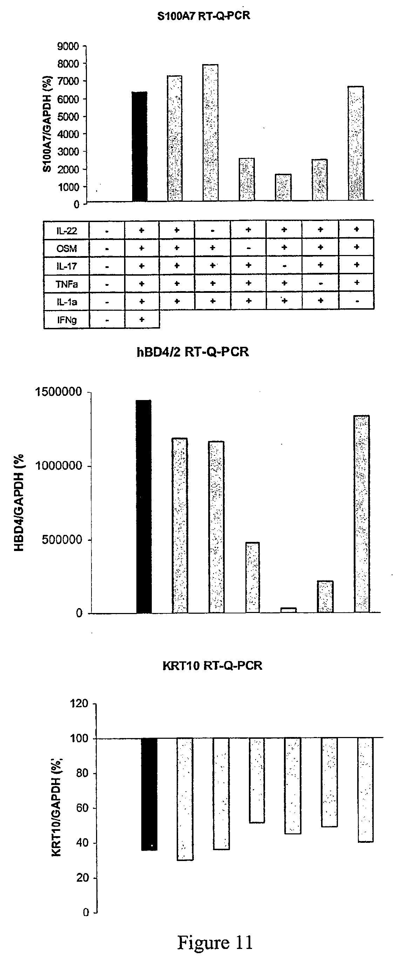

The results are shown in Table 1 below and in Figure 11.

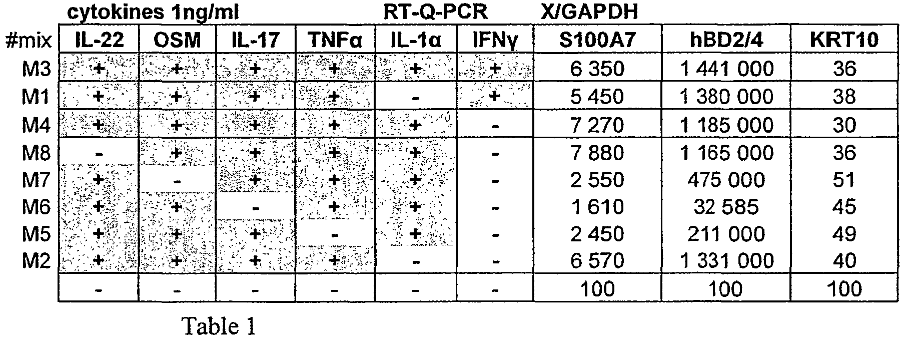

The mix of the 6 selected cytokines (M3) exhibited a strong synergic effect on the expression of the keratinocyte inflammation markers psoriasin (S100A7) and defensin beta-2/beta-4 (hBD2/4). The depletion of the mixes in either IFNγ, IL-22, or IL-1α, did not significantly decrease the activity of the complete cocktail of cytokines; these cytokines are hence probably not directly involved in the observed synergy. To the contrary,
- the omission of OSM from the 5 cytokines reference mix (IFNγ has been omitted because it is inactive) led to a decrease of the activity of the mix by 3-fold for S100A7 and by 2.5-fold for hBD2/4;
- the omission of TNFα led to a decrease of the activity of the mix by 3-fold for S100A7 and by 5.6-fold for hBD2/4; and
- the omission of IL-17 led to a decrease of the activity of the mix by 4.5-fold for S100A7 and by 36-fold for hBD2/4.

These results indicate a strong synergy between OSM, TNFα and IL-17 for a maximal response in keratinocytes.

Hence, it appears that OSM is able to synergize with IL-17 and/or with TNFα in pathologic situations such as psoriasis and that the blockage of the signalling of these two cytokines may lead to a dramatic inhibition of the physiopathologic response of the target keratinocytes, allowing to a reversion of the pathology.

### EXAMPLE 12: Involvement of the type II OSM receptor and OSM the pathogenesis of psoriasis

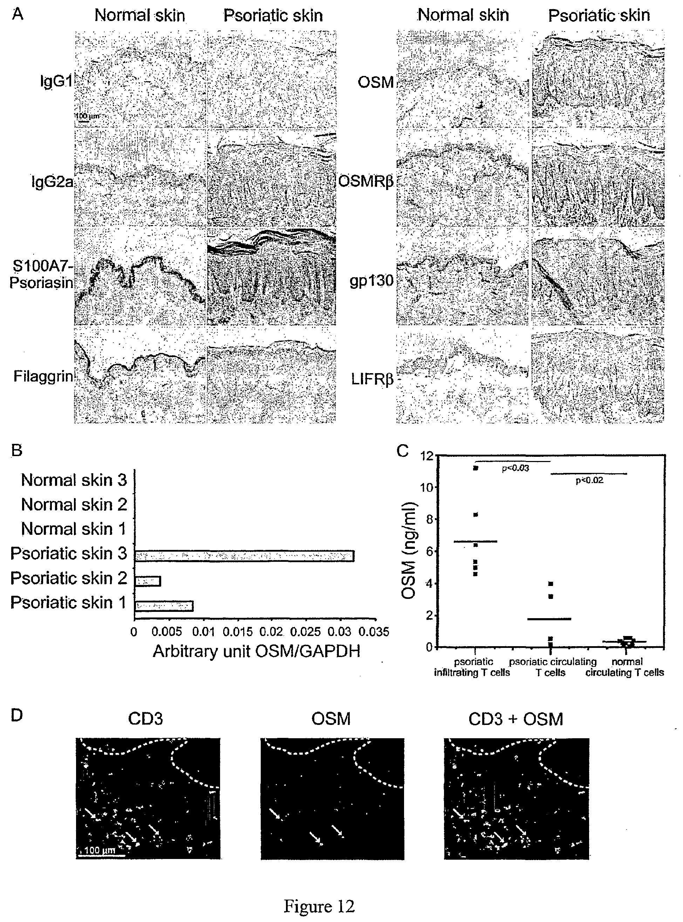

Since OSM is a potent inducer of keratinocyte motility and triggers hyperplasia of RHE, the expression of the OSMRβ, LIFRβ and gp130 receptor subunits in active cutaneous lesions from psoriatic patients was analyzed by immunohistochemistry. This type of lesions is characterized by a thickened epidermis, a decreased stratum granulosum, parakeratosis and a strong up-regulation of S100A7-psoriasin expression, together with a concomitant decrease in the expression of filaggrin (Fig. 12A). Both OSMRβ and gp130 were highly expressed in psoriatic, as compared to normal skin (Fig. 12A). The expression of LIFRβ was undetectable (Fig. 12A), in agreement with the above results obtained on NHEK. Taken together, these results indicate that under conditions of cutaneous inflammation, the expression of the type II OSMR is up-regulated on human keratinocytes and furthermore confirm the absence of the type I OSMR in both healthy and psoriatic skin.

Concomitantly, OSM mRNA and protein were strongly expressed in psoriatic lesions as compared to normal skin where no signal could be detected (Fig. 12A and 12B), in line with the previously reported release of OSM by short term culture of psoriatic skin (Bonifati et al., 1998).

### EXAMPLE 13: OSM production by lesion-infiltrating T cells and, to a lower extend, by keratinocytes

In order to extend the observation of example 12, the inventors tested the possibility whether skin-infiltrating T cells could be a source of OSM in these lesions. Indeed, as shown in Figure 12D, a subset of CD3⁺ T lymphocytes were OSM producers. This observation was confirmed in anti-CD3/CD28 mAb activated T cells isolated and expanded from the psoriatic lesion that were found to produce high levels of OSM, as compared to those obtained with peripheral blood T cells from the same patients or from healthy donors (Fig. 12C). Nonetheless, residual anti-OSM reactivity was detected in lesional keratinocyte themselves both by immunoenzymatic and immunofluorescent stainings (Fig. 12A and 12D). To precise this point, OSM mRNA was measured by quantitative RT-PCR in NHEK, cultured in the presence or absence of OSM or culture supernatants from activated psoriatic skin-infiltrating T cells. These results showed that control or stimulated NHEK express 20 to 100 fold less OSM mRNA than in activated T cells derived from psoriatic skin. Finally, the inventors failed to detect any OSM production by ELISA in NHEK supernatants, indicating that keratinocytes themselves synthesized very limited amounts of the cytokine. Furthermore, no circulating OSM could be detected in the sera of these patients. Taken together, these results demonstrate that T cells infiltrating the psoriatic lesions are a major source of OSM and are likely to contribute, *via* the induction of keratinocyte inflammation, to the pathogenesis of psoriasis.

### EXAMPLE 14: OSM induces a psoriasis-like phenotype in mice

To analyze the direct contribution of OSM to the pathogenesis of psoriasis, intradermal injections of OSM were performed in mice. After 4 days of treatment, the site of injection showed a 2 to 3 fold epidermal thickening in OSM-treated mice, as compared to animals treated with vehicle alone (Fig. 13A). Further characterization of these skin samples by quantitative RT-PCR analysis showed that OSM enhances the expression of S100A8, S100A9, MIP-1β and macrophage-derived chemokine (MDC) genes (Fig. 13B). These data support the notion that OSM is an important mediator directly involved in epidermal chemotaxis, thickening and inflammation of the skin.

### REFERENCES

Alowami, S., G. Qing, et al. (2003). "Psoriasin (S100A7) expression is altered during skin tumorigenesis." BMC Dermatol 3(1): 1.
Benigni, F., G. Fantuzzi, et al. (1996). "Six different cytokines that share GP130 as a receptor subunit, induce serum amyloid A and potentiate the induction of interleukin-6 and the activation of the hypothalamus-pituitary-adrenal axis by interleukin-1." Blood 87(5): 1851-4.
Bernard, F. X., N. Pedretti, et al. (2002). "Comparison of gene expression profiles in human keratinocyte mono-layer cultures, reconstituted epidermis and normal human skin; transcriptional effects of retinoid treatments in reconstituted human epidermis." Exp Dermatol 11(1): 59-74.
Bonifati, C., A. Mussi, et al. (1998). "spontaneous release of leukemia inhibitory factor and oncostatin-M is increased in supernatants of short-term organ cultures from lesional psoriatic skin." Arch Dermatol Res 290(1-2): 9-13.
Boulton, T. G., N. Stahl, et al. (1994). "Ciliary neurotrophic factor/leukemia inhibitory factor/interleukin 6/oncostatin M family of cytokines induces tyrosine phosphorylation of a common set of proteins overlapping those induced by other cytokines and growth factors." J Biol Chem 269(15): 11648-55.
Broome, A. M., D. Ryan, et al. (2003). "S100 protein subcellular localization during epidermal differentiation and psoriasis." J Histochem Cytochem 51(5): 675-85.
Bullard, K. M., L. Lund, et al. (1999). "Impaired wound contraction in stromelysin-1-deficient mice." Ann Surg 230(2): 260-5.
Dillon, S. R., C. Sprecher, et al. (2004). "Interleukin 31, a cytokine produced by activated T cells, induces dermatitis in mice." Nat Immunol 5(7): 752-60.
Diveu, C., A. H. Lak-Hal, et al. (2004). "Predominant expression of the long isoform of GP130-like (GPL) receptor is required for interleukin-31 signaling." Eur Cytokine Netw 15(4): 291-302.
Donato, R. (1999). "Functional roles of S 100 proteins, calcium-binding proteins of the EF-hand type." Biochim Biophys Acta 1450(3): 191-231.
Gallucci, R. M., P. P. Simeonova, et al. (2000). "Impaired cutaneous wound healing in interleukin-6-deficient and immunosuppressed mice." Faseb J 14(15): 2525-31.
Gallucci, R. M., D. K. Sloan, et al. (2004). "Interleukin 6 indirectly induces keratinocyte migration." J Invest Dermatol 122(3): 764-72.
Gebhardt, C., U. Breitenbach, et al. (2002). "Calgranulins S100A8 and S100A9 are negatively regulated by glucocorticoids in a c-Fos-dependent manner and overexpressed throughout skin carcinogenesis." Oncogene 21(27): 4266-76.
Ihn, H. and K. Tamaki (2000). "Oncostatin M stimulates the growth of dermal fibroblasts via a mitogen-activated protein kinase-dependent pathway." J Immunol 165(4): 2149-55.
Jonak, C., G. Klosner, et al. (2002). "Subcorneal colocalization of the small heat shock protein, hsp27, with keratins and proteins of the cornified cell envelope." Br J Dermatol 147(1): 13-9.
Kira, M., S. Sano, et al. (2002). "STAT3 deficiency in keratinocytes leads to compromised cell migration through hyperphosphorylation of p130(cas)." J Biol Chem 277(15): 12931-6.
Koch, A. E., S. L. Kunkel, et al. (1994). "Epithelial neutrophil activating peptide-78: a novel chemotactic cytokine for neutrophils in arthritis." J Clin Invest 94(3): 1012-8.
Komine, M., L. S. Rao, et al. (2000). "Inflammatory versus proliferative processes in epidermis. Tumor necrosis factor alpha induces K6b keratin synthesis through a transcriptional complex containing NFkappa B and C/EBPbeta." J Biol Chem 275(41): 32077-88.
Lew, W., A. M. Bowcock, et al. (2004). "Psoriasis vulgaris: cutaneous lymphoid tissue supports T-cell activation and "Type 1" inflammatory gene expression." Trends Immunol 25(6):295-305.
Lugering, N., T. Kucharzik, et al. (1997). "Importance of combined treatment with IL-10 and IL-4, but not IL-13, for inhibition of monocyte release of the Ca(2+)-binding protein MRP8/14." Immunology 91(1): 130-4.
Madsen, P., H. H. Rasmussen, et al. (1991). "Molecular cloning, occurrence, and expression of a novel partially secreted protein "psoriasin" that is highly up-regulated in psoriatic skin." J Invest Dermatol 97(4): 701-12.
Mehul, B., D. Bernard, et al. (2001). "Calmodulin-like skin protein: a new marker of keratinocyte differentiation." J Invest Dermatol 116(6): 905-9.
Mehul, B., D. Bernard, et al. (2000). "Identification and cloning of a new calmodulin-like protein from human epidermis." J Biol Chem 275(17): 12841-7.
Nagase, H. and J. F. Woessner, Jr. (1999). "Matrix metalloproteinases." J Biol Chem 274(31): 21491-4.
Navarro, J. M., J. Casatorres, et al. (1995). "Elements controlling the expression and induction of the skin hyperproliferation-associated keratin K6." J Biol Chem 270(36): 21362-7.
Paglia, D., S. Kondo, et al. (1996). "Leukaemia inhibitory factor is expressed by normal human keratinocytes in vitro and in vivo." Br J Dermatol 134(5): 817-23.
Paramio, J. M., M. L. Casanova, et al. (1999). "Modulation of cell proliferation by cytokeratins K10 and K16." Mol Cell Biol 19(4): 3086-94.
Pattyn, F., F. Speleman, et al. (2003). "RTPrimerDB: the real-time PCR primer and probe database." Nucleic Acids Res 31(1): 122-3.
Pfaffl, M. W. (2001). "A new mathematical model for relative quantification in real-time RT-PCR." Nucleic Acids Res 29(9): e45.
Pilcher, B. K., M. Wang, et al. (1999). "Role of matrix metalloproteinases and their inhibition in cutaneous wound healing and allergic contact hypersensitivity." Ann N Y Acad Sci 878: 12-24.
Rogers, M. S., T. Kobayashi, et al. (2001). "Human calmodulin-like protein is an epithelial-specific protein regulated during keratinocyte differentiation." Exp Cell Res 267(2): 216-24.
Rollman, O., U. B. Jensen, et al. (2003). "Platelet derived growth factor (PDGF) responsive epidermis formed from human keratinocytes transduced with the PDGF beta receptor gene." J Invest Dermatol 120(5): 742-9.
Rosdy, M., B. Bertino, et al. (1997). "Retinoic acid inhibits epidermal differentiation when applied topically on the stratum corneum of epidermis formed in vitro by human keratinocytes grown on defined medium." In Vitro Toxicology 10(1): 39-47.
Roth, J., T. Vogl, et al. (2003). "Phagocyte-specific S100 proteins: a novel group of proinflammatory molecules." Trends Immunol 24(4): 155-8.
Ryckman, C., K. Vandal, et al. (2003). "Proinflammatory activities of S100: proteins S100A8, S100A9, and S100A8/A9 induce neutrophil chemotaxis and adhesion." J Immunol 170(6): 3233-42.
Sano, S., K. S. Chan, et al. (2005). "Stat3 links activated keratinocytes and immunocytes required for development of psoriasis in a novel transgenic mouse model." Nat Med 11(1): 43-9.
Sano, S., S. Itami, et al. (1999). "Keratinocyte-specific ablation of Stat3 exhibits impaired skin remodeling, but does not affect skin morphogenesis." Embo J 18(17): 4657-68.
Sugawara, T., R. M. Gallucci, et al. (2001). "Regulation and role of interleukin 6 in wounded human epithelial keratinocytes." Cytokine 15(6): 328-36.
Taga, T. and T. Kishimoto (1997). "Gp130 and the interleukin-6 family of cytokines." Annu Rev Immunol 15: 797-819.
Thorey, I. S., J. Roth, et al. (2001). "The Ca2+-binding proteins S100A8 and S100A9 are encoded by novel injury-regulated genes. "J Biol Chem 276(38): 35818-25.
Trickett, A. and Y. L. Kwan (2003). "T cell stimulation and expansion using anti-CD3/CD28 beads." J Immunol Methods 275(1-2): 251-5.
Vandesompele, J., K. De Preter, et al. (2002). "Accurate normalization of real-time quantitative RT-PCR data by geometric averaging of multiple internal control genes." Genome Biol 3(7): RESEARCH0034.
Wagener, F. A., H. E. van Beurden, et al. (2003). "The heme-heme oxygenase system: a molecular switch in wound healing." Blood 102(2): 521-8.
Wahl, A. F. and P. M. Wallace (2001). "Oncostatin M in the anti-inflammatory response." Ann Rheum Dis 60 Suppl 3: iii75-80.
Watson, P. H., E. R. Leygue, et al. (1998). "Psoriasin (S100A7)." Int J Biochem Cell Biol 30(5): 567-71.
Yssel, H., S. Lecart, et al. (2001). "Regulatory T cells and allergic asthma." Microbes Infect 3(11): 899-904.
Yu, J. Y., S. L. DeRuiter, et al. (2002). "RNA interference by expression of short-interfering RNAs and hairpin RNAs in mammalian cells." Proc Natl Acad Sci U S A 99(9): 6047-52.

## Claims

1. Use of at least one molecule selected in the group consisting of an OSM antagonist selected from a neutralizing monoclonal antibody directed against OSM (oncostatin M) or against the OSM receptor, a modified OSM, a soluble OSM receptor, a decoy OSM receptor and OSM expression inhibitors, for the preparation of a composition for reducing and/or treating keratinocyte-mediated inflammation.

2. The use according to claim 1, wherein said expression inhibitor is a siRNA.

3. The use according to any of claim 1 to 3, for the preparation of a composition for preventing, alleviating or treating a skin inflammation disease selected in the group consisting of psoriasis, atopic dermatitis, bullous epidermolysis, bullous phemphigoid, lichen, acne, eczema, professional dermatitis, seborrheic dermatitis, keratosis, rosacea, erythema and ichtyosis.

4. The use according to any of claims 1 to 4, wherein said composition is used for inhibiting keratinocyte migration.

5. The use according to any of claims 1 to 5, wherein said composition is used for preventing or reducing epidermal thickening.

6. The use according to any of claims 1 to 6, wherein said composition is used for slowing down epidermal healing.

7. The use according to any of claims 1 to 7, wherein said composition is used for preventing and/or attenuating chaps on hands, lips, face or body.

8. The use according to any of claims 1 to 8, wherein said composition is used for preventing and/or attenuating stretch marks.

9. The use according to any of claims 1 to 9, wherein said composition is used for improving the aspect and comfort of scars.

10. The use according to any of claims 1 to 10, wherein said composition is used for improving the aspect and comfort of epidermal wounds during their cicatrisation.

11. The use according to any of claims 1 to 11, for the preparation of a composition for topic administration.

12. The use according to any of claims 1 to 12, wherein said antagonist and/or an expression inhibitor of OSM is used in combination with at least one additional molecule selected in the group consisting of antagonists and expression inhibitors of a cytokine selected amongst IL-17, TNFα, IL-31 and IFN-γ, and antagonists and expression inhibitors of the OSMRβ subunit.

13. The use according to any of claims 1 to 13, wherein the composition is a mix containing, in addition to an antagonist and/or an expression inhibitor of OSM, at least one additional molecule selected in the group consisting of antagonists and expression inhibitors of a cytokine selected amongst IL-17, TNFα, IL-31 and IFN-γ, and antagonists and expression inhibitors of the OSMRβ subunit.

14. A cosmetic and/or dermatological composition **characterized in that** it comprises an antagonist of OSM selected from a neutralizing monoclonal antibody directed against OSM (oncostatin M) or against the OSM receptor, a modified OSM, a soluble OSM receptor, a decoy OSM receptor and OSM expression inhibitors.

15. The cosmetic and/or dermatological composition according to claim 15, **characterized in that** it further comprises an antagonist of IL-17.

16. The cosmetic and/or dermatological composition according to claim 15 or claim 16, **characterized in that** it further comprises an antagonist of TNFα.

17. A method for preparing an *in vitro* model of psoriasis, **characterized in that** it comprises a step of adding OSM to cultured keratinocytes.

18. The method of claim 18, further comprising a step of adding IL-17 and/or TNFα to said cultured keratinocytes.

19. The method according to claim 18 or 19, wherein OSM is added in a concentration ranging 0.1 to 100 ng/ml.

20. A method for obtaining an animal model of psoriasis to the exclusion of humans, **characterized in that** it comprises a step of administering OSM to said animal.

21. The method of claim 21, further comprising a step of administering IL-17 and/or TNFα to said animal.

22. The method according to claim 21 or 22, wherein OSM is administered, topically and/or subcutaneously and/or via intradermal injection.

23. Use of a model of psoriasis obtained through the method according to any of claims 18 to 23, for the screening of molecules.

24. Use according to claim 24, for the identification of molecules having anti-inflammatory properties at the skin level.

## Patentansprüche

1. Verwendung mindestens eines Moleküls, ausgewählt aus der Gruppe, bestehend aus einem OSM-Antagonisten, ausgewählt aus einem neutralisierenden monoklonalen Antikörper gegen OSM (Oncostatin M) oder gegen den OSM-Rezeptor, einem modifizierten OSM-, einem löslichen OSM-Rezeptor, einem OSM-Köderrezeptor und OSM-Expressionsinhibitoren in der Herstellung einer Zusammensetzung zur Linderung und/oder Behandlung Keratinozyt-vermittelter Entzündung.

2. Verwendung gemäß Anspruch 1, wobei der Expressionsinhibitor eine siRNA ist.

3. Verwendung gemäß einem der Ansprüche 1 bis 3 für die Herstellung einer Zusammensetzung zur Vorbeugung, Linderung oder Behandlung einer Hautentzündungserkrankung, ausgewählt aus der Gruppe, bestehend aus Psoriasis, atopischer Dermatitis, Epidermolysis bullosa, bullösem Phemphigoid, Flechte, Akne, Ekzem, beruflich bedingter Dermatitis, seborrhoischer Dermatitis, Keratose, Rosacea, Erythem und Ichtyose.

4. Verwendung gemäß einem der Ansprüche 1 bis 4, wobei die Zusammensetzung zur Inhibierung der Keratinozytenmigration verwendet wird.

5. Verwendung gemäß einem der Ansprüche 1 bis 5, wobei die Zusammensetzung zur Vorbeugung oder Linderung von Epidermis-Verdickung verwendet wird.

6. Verwendung gemäß einem der Ansprüche 1 bis 6, wobei die Zusammensetzung zur Verlangsamung der Epidermis verwendet wird.

7. Verwendung gemäß einem der Ansprüche 1 bis 7, wobei die Zusammensetzung zur Vorbeugung und/oder Linderung von Rissen an Händen, Lippen, dem Gesicht oder dem Körper verwendet wird.

8. Verwendung gemäß einem der Ansprüche 1 bis 8, wobei die Zusammensetzung zur Vorbeugung und/oder Linderung von Schwangerschaftsstreifen verwendet wird.

9. Verwendung gemäß einem der Ansprüche 1 bis 9, wobei die Zusammensetzung zur Verbesserung des Aussehens und Ertragens von Narben verwendet wird.

10. Verwendung gemäß einem der Ansprüche 1 bis 10, wobei die Zusammensetzung zur Verbesserung des Aussehens und Ertragens epidermischer Wunden während deren Vernarbung verwendet wird.

11. Verwendung gemäß einem der Ansprüche 1 bis 11 für die Herstellung einer Zusammensetzung zur topischen Verabreichung.

12. Verwendung gemäß einem der Ansprüche 1 bis 12, wobei der Antagonist und/oder ein Expressionsinhibitor von OSM in Kombination mit mindestens einem zusätzlichen Molekül, ausgewählt aus der Gruppe, bestehend aus Antagonisten und Expressionsinhibitoren eines Zytokins, ausgewählt aus IL-17, TNFα, IL-31 und IFN-γ, und Antagonisten und Expressionsinhibitoren der OSMRβ-Untereinheit, verwendet wird.

13. Verwendung gemäß einem der Ansprüche 1 bis 13, wobei die Zusammensetzung eine Mischung ist, die zusätzlich zu einem Antagonisten und/oder einem Expressionsinhibitor von OSM mindestens ein zusätzliches Molekül enthält, ausgewählt aus der Gruppe, bestehend aus Antagonisten und Expressionsinhibitoren eines Zytokins, ausgewählt aus IL-17, TNFα, IL-31 und IFN-γ, und Antagonisten und Expressionsinhibitoren der OSMRβ-Untereinheit.

14. Kosmetikum und/oder dermatologische Zusammensetzung, **dadurch gekennzeichnet, dass** es bzw. sie einen OSM-Antagonisten, ausgewählt aus einem neutralisierenden monoklonalen Antikörper gegen OSM (Oncostatin M) oder gegen den OSM-Rezeptor, einem modifizierten OSM, einem löslichen OSM-Rezeptor, einem OSM-Köderrezeptor und OSM-Expressionsinhibitoren, umfasst.

15. Kosmetikum und/oder dermatologische Zusammensetzung gemäß Anspruch 15, **dadurch gekennzeichnet, dass** es bzw. sie des weiteren einen Antagonisten von IL-17 umfasst.

16. Kosmetikum und/oder dermatologische Zusammensetzung gemäß Anspruch 15 oder Anspruch 16, **dadurch gekennzeichnet, dass** es bzw. sie des weiteren einen Antagonisten von TNFα umfasst.

17. Verfahren zur Herstellung eines in vitro-Modells von Psoriasis, **dadurch gekennzeichnet, dass** es einen Schritt der Zugabe von OSM zu kultivierten Keratinozyten umfasst.

18. Verfahren gemäß Anspruch 18, des weiteren umfassend einen Schritt der Zugabe von IL-17 und/oder TNFα zu den kultivierten Keratinozyten.

19. Verfahren gemäß Anspruch 18 oder 19, wobei OSM in einer Konzentration, die von 0,1 bis 100 ng/ml reicht, zugegeben wird.

20. Verfahren zum Erhalt eines Tiermodells von Psoriasis unter Ausschluss von Menschen, **dadurch gekennzeichnet, dass** es einen Schritt der Verabreichung von OSM an das Tier umfasst.

21. Verfahren gemäß Anspruch 21, des weiteren umfassend einen Schritt der Verabreichung von IL-17 und/oder TNFα an das Tier.

22. Verfahren gemäß Anspruch 21 oder 22, wobei OSM topisch und/oder subkutan und/oder über intradermale Injektion verabreicht wird.

23. Verwendung eines Psoriasis-Modells, erhalten durch das Verfahren gemäß einem der Ansprüche 18 bis 23, zum Screening von Molekülen.

24. Verwendung gemäß Anspruch 24 zur Identifizierung von Molekülen mit anti-inflammatorischen Eigenschaften auf der Hautebene.

## Revendications

1. Utilisation d'au moins une molécule sélectionnée dans le groupe consistant en un antagoniste de l'OSM sélectionné parmi un anticorps monoclonal neutralisant dirigé contre l'OSM (oncostatine M) ou contre le récepteur de l'OSM, une OSM modifiée, un récepteur soluble de l'OSM, un récepteur leurre de l'OSM et des inhibiteurs de l'expression de l'OSM, pour la préparation d'une composition destinée à réduire et/ou traiter une inflammation médiée par les kératinocytes.

2. Utilisation selon la revendication 1, dans laquelle ledit inhibiteur de l'expression est un ARNsi.

3. Utilisation selon l'une quelconque des revendications 1 à 3, pour la préparation d'une composition destinée à prévenir, soulager ou traiter une maladie inflammatoire cutanée sélectionnée dans le groupe constitué du psoriasis, de la dermatite atopique, de l'épidermolyse bulleuse, de la pemphigoïde bulleuse, du lichen, de l'acné, de l'eczéma, d'une dermatite professionnelle, de la dermatite séborrhéique, de la kératose, de la rosacée, d'un érythème et d'une ichtyose.

4. Utilisation selon l'une quelconque des revendications 1 à 4, dans laquelle ladite composition est utilisée pour inhiber la migration des keratinocytes.

5. Utilisation selon l'une quelconque des revendications 1 à 5, dans laquelle ladite composition est utilisée pour prévenir ou réduire l'épaississement de l'épiderme.

6. Utilisation selon l'une quelconque des revendications 1 à 6, dans laquelle ladite composition est utilisée pour ralentir la cicatrisation de l'épiderme.

7. Utilisation selon l'une quelconque des revendications 1 à 7, dans laquelle ladite composition est utilisée pour prévenir et/ou atténuer les gerçures sur les mains, les lèvres, le visage ou le corps.

8. Utilisation selon l'une quelconque des revendications 1 à 8, dans laquelle ladite composition est utilisée pour prévenir et/ou atténuer les vergetures.

9. Utilisation selon l'une quelconque des revendications 1 à 9, dans laquelle ladite composition est utilisée pour améliorer l'aspect et le confort des cicatrices.

10. Utilisation selon l'une quelconque des revendications 1 à 10, dans laquelle ladite composition est utilisée pour améliorer l'aspect et le confort de plaies épidermiques durant leur cicatrisation.

11. Utilisation selon l'une quelconque des revendications 1 à 11, pour la préparation d'une composition destinée à une administration topique.

12. Utilisation selon l'une quelconque des revendications 1 à 12, dans laquelle ledit antagoniste et/ou ledit inhibiteur de l'expression de l'OSM est utilisé en combinaison avec au moins une molécule supplémentaire sélectionnée dans le groupe constitué d'antagonistes et d'inhibiteurs de l'expression d'une cytokine sélectionnée parmi l'IL-17, le TNFα, l'IL-31, et l'IFNγ, et les antagonistes et inhibiteurs de l'expression de la sous-unité OSMRβ.

13. Utilisation selon l'une quelconque des revendications 1 à 13, dans laquelle la composition est un mélange contenant, outre un antagoniste et/ou un inhibiteur de l'expression de l'OSM, au moins une molécule supplémentaire sélectionnée dans le groupe constitué d'antagonistes et d'inhibiteurs de l'expression d'une cytokine sélectionnée parmi l'IL-17, le TNFα, l'IL-31, et l'IFNγ, et des antagonistes et inhibiteurs de l'expression de la sous-unité OSMRβ.

14. Composition cosmétique et/ou dermatologique **caractérisée en ce qu'**elle comprend un antagoniste de l'OSM, sélectionné parmi un anticorps monoclonal neutralisant dirigé contre l'OSM (oncostatine M) ou contre le récepteur de l'OSM, une OSM modifiée, un récepteur soluble de l'OSM, un récepteur leurre de l'OSM et des inhibiteurs de l'expression de l'OSM.

15. Composition cosmétique et/ou dermatologique selon la revendication 15, **caractérisée en ce qu'**elle comprend en outre un antagonisme de l'IL-17.

16. Composition cosmétique et/ou dermatologique selon la revendication 15 ou la revendication 16, **caractérisée en ce qu'** elle comprend en outre un antagoniste du TNFα.

17. Procédé de préparation d'un modèle *in vitro* de psoriasis **caractérisé en ce qu'**il comprend une étape d'ajout d'OSM à des kératinocytes en culture.

18. Procédé selon la revendication 18, comprenant en outre une étape d'ajout d'IL-17 et/ou de TNFα auxdits keratinocytes en culture.

19. Procédé selon la revendication 18 ou 19, dans lequel l'OSM est ajoutée en une concentration dans la plage de 0,1 à 100 ng/mL.

20. Procédé d'obtention d'un modèle animal de psoriasis à l'exclusion des humains, **caractérisé en ce qu'**il comprend une étape d'administration d'OSM au dit animal.

21. Procédé selon la revendication 21, comprenant en outre une étape d'administration d'IL-17 et/ou de TNFα au dit animal.

22. Procédé selon la revendication 21 ou 22, dans lequel l'OSM est administrée par voie topique et/ou sous-cutanée et/ou par injection intradermique.

23. Utilisation d'un modèle de psoriasis obtenu par le procédé selon l'une quelconque des revendications 18 à 23, pour le criblage de molécules.

24. Utilisation selon la revendication 24, pour l'identification de molécules ayant des propriétés anti-inflammatoires au niveau cutané.
